# EUROPEAN PATENT SPECIFICATION

(11) **EP 1 996 208 B1**
(45) Date of publication and mention of the grant of the patent: **25.09.2013**
(21) Application number: 07763709.8
(22) Date of filing: 09.02.2007
(51) Int. Cl.: A61K 31/765, A61K 31/785, A61K 9/14, A61K 47/48, A61P 35/00

(54) **Multi-arm polymeric conjugates of 7-ethyl-10-hydroxycamptothecin for treatment of breast, colorectal, pancreatic, ovarian and lung cancers**
Mehrarmige Polymerkonjugate von 7-Ethyl-10-Hydroxycamptothecin zur Behandlung von Brust-, Kolorektal-, Bauchspeicheldrüsen-, Ovarial- und Lungenkrebs
Conjugués polymériques de 7-éthyl-10-hydroxycamptothécine à bras multiples utilisés dans le traitement du cancer du sein, du cancer colorectal, du cancer du pancréas, de l'ovaire et du poumon

(30) Priority: 09.02.2006 US 772464 P; 09.06.2006 US 804391 P; 15.09.2006 US 844938 P; 06.11.2006 US 864516 P
(43) Date of publication of application: 03.12.2008
(73) Proprietor: Belrose Pharma Inc., Princeton, NJ 08540 (US)
(72) Inventor: ZHAO, Hong, Edison, NJ 08820 (US); RUBIO, Maria, Belen, Somerset, NJ 08873 (US); SAPRA, Puja, Edison, NJ 08820 (US); WU, Dechun, Bridgewater, NJ 08807 (US)
(74) Representative: Hatzmann, Martin
(86) International application number: PCT/US2007/003808
(87) International publication number: WO 2007/092646

(56) References cited:
- WO-A1-96/23794
- WO-A2-01/68066
- US-A1- 2004 009 229
- US-A1- 2004 247 624
- US-A1- 2005 112 088
- ZHAO H ET AL: "Novel prodrugs of SN38 generated by multi-arm poly(ethylene glycol)", EJC SUPPLEMENTS, vol. 4, no. 12, November 2006 (2006-11), page 50, XP002624208, & 18TH SYMPOSIUM ON MOLECULAR TARGETS AND CANCER THERAPEUTICS; PRAGUE, CZECH REPUBLIC; NOVEMBER 07 -10, 2006 ISSN: 1359-6349
- CONOVER C D ET AL: "CAMPTOTHECIN DELIVERY SYSTEMS: ENHANCED EFFICACY AND TUMOR ACCUMULATION OF CAMPTOTHECIN FOLLOWING ITS CONJUGATION TO POLYETHYLENE GLYCOL VIA A GLYCINE LINKER", CANCER CHEMOTHERAPY AND PHARMACOLOGY, SPRINGER VERLAG, BERLIN, vol. 42, no. 5, 1 January 1998 (1998-01-01), pages 407-414, XP001023467, ISSN: 0344-5704, DOI: DOI:10.1007/S002800050837
- GREENWALD R B ET AL: "CAMPTOTHECIN-20-PEG ESTER TRANSPORT FORMS: THE EFFECT OF SPACER GROUPS ON ANTITUMOR ACTIVITY", BIOORGANIC & MEDICINAL CHEMISTRY, PERGAMON, GB, vol. 6, 1 January 1998 (1998-01-01), pages 551-562, XP000985272, ISSN: 0968-0896, DOI: DOI:10.1016/S0968-0896(98)00005-4
- YU ET AL: "Antitumor activity of poly(ethylene glycol)-camptothecin conjugate: The inhibition of tumor growth in vivo", JOURNAL OF CONTROLLED RELEASE, ELSEVIER, AMSTERDAM, NL, vol. 110, no. 1, 10 December 2005 (2005-12-10), pages 90-102, XP005192555, ISSN: 0168-3659, DOI: DOI:10.1016/J.JCONREL.2005.09.050
- LUTOLF M P ET AL: "Synthesis and physicochemical characterization of end-linked poly(ethylene glycol)-co-peptide hydrogels formed by Michael-type addition", BIOMACROMOLECULES, ACS, WASHINGTON, DC, US, vol. 4, no. 3, 26 February 2003 (2003-02-26), pages 713-722, XP002390066, ISSN: 1525-7797, DOI: DOI:10.1021/BM025744E
- ZHAO HONG ET AL: "Novel prodrugs of SN38 using multiarm poly(ethylene glycol) linkers.", BIOCONJUGATE CHEMISTRY APR 2008 LNKD- PUBMED:18370417, vol. 19, no. 4, April 2008 (2008-04), pages 849-859, XP002624209, ISSN: 1520-4812

## Description

### CROSS-REFERENCE TO RELATED APPLICATION

This application claims the benefit of priority from U.S. Provisional Patent Application Serial Nos. 60/772,464 filed February 9, 2006, 60/804,391 filed June 9, 2006, 60/844,938 filed September 15, 2006 and 60/864,516 filed November 6, 2006.

### FIELD OF INVENTION

The present invention relates to multi-arm polymeric prodrugs of 7-ethyl-10-hydroxycamptothecin. In particular, the invention relates to four-arm polyethylene glycol conjugates of 7-ethyl-10-hydroxycamptothecin and use thereof.

### BACKGROUND OF INVENTION

Over the years, several methods of administering biologically-effective materials to mammals have been proposed. Problems arise when the desired medicinal agent is insoluble in aqueous fluids. Alkaloids are often especially difficult to solubilize.

Camptothecin is a water-insoluble cytotoxic alkaloid produced by *Camptotheca accuminata* trees indigenous to China and *Nothapodytes foetida* trees indigenous to India. Camptothecin and related analogs are known to be potential anticancer agents and have demonstrated therapeutic activity *in vitro* and *in vivo.*

Camptothecin and analogs are known as DNA topoisomerase I inhibitors. For example, one of camptothecin analogs is Irinotecan (CPT-11, Camptosar®) which is also a DNA topoisomerase I inhibitor and has also showed anticancer activity. 7-ethyl-10-hydroxycampothecin is an active metabolite of CPT-11.

Document US 2005/0112088 discloses multi-arm PEG prodrug conjugates of a camptothecin compound. Said prodrugs are water-soluble and exhibit improved properties in-vivo when compared to the unmodified parent camptothecin.

### SUMMARY OF INVENTION

In order to overcome the above problems and improve the technology for drug delivery through PEGylation, there are provided new methods to directly use multi-armed PEGs to conjugate to drug molecules through proper linkers. In this way, there is no need to incorporate laborious branching moieties to the PEG. In addition, the drug molecules are relatively far from each other. Therefore the conjugation efficiency to the PEG is greatly increased. The solubility of the PEG conjugate is also improved over the endbranched approach.

In one aspect of the invention, compounds of Formula (I) are provided: 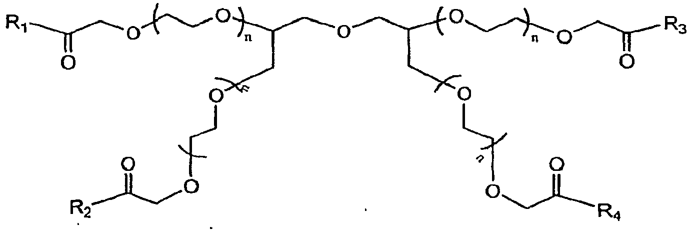 wherein
R₁, R₂, R₃ and R₄ are independently OH or (L)ₘ-D;
L is a bifunctional linker;
D is a residue of a camptothecin or a camptothecin analog, such as 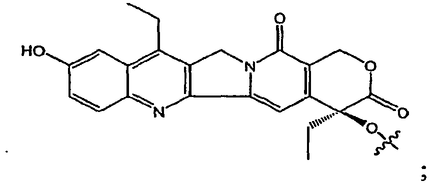
m is a positive integer from 1 to 10;
n is a positive integer;
provided that R₁, R₂, R₃ and R₄ are not all OH.

In this aspect of the invention, the camptothecin or camptothecin analog is reacted with the polymer so that the 20-OH group thereof forms conjugates with the four-arm polymers.

In one preferred aspect of the invention, L is a residue of an amino acid. In certain preferred aspects of the invention, n is from 28 to 341, and is preferably 227.

Methods of making the conjugates as well as compounds of the present invention for use in therapy are also provided.

Advantages will be apparent from the following description and drawings.

For purposes of the present invention, the term "residue" shall be understood to mean that portion of a compound, to which it refers, i.e. camptothecin analog, 7-ethyl-10-hydroxycamptothecin, amino acid, etc. that remains after it has undergone a substitution reaction with another compound.

For purposes of the present invention, the term "polymeric containing residue" or "PEG residue" shall each be understood to mean that portion of the polymer or PEG which remains after it has undergone a reaction with camptothecin or camptothecin analog-containing compounds.

For purposes of the present invention, the term "alkyl" shall be understood to include straight, branched, substituted, e.g. halo-, alkoxy-, nitro-, C₁₋₁₂, but preferably C₁₋₄ alkyls, C₃₋₈ cycloalkyls or substituted cycloalkyls.

For purposes of the present invention, the term "substituted" shall be understood to include adding or replacing one or more atoms contained within a functional group or compound with one or more different atoms.

For purposes of the present invention, substituted alkyls include carboxyalkyls, aminoalkyls, dialkylaminos, hydroxyalkyls and mercaptoalkyls; substituted alkenyls include carboxyalkenyls, aminoalkenyls, dialkenylaminos, hydroxyalkenyls and mercaptoalkenyls; substituted alkynyls include carboxyalkynyls, aminoalkynyls, dialkynylaminos, hydroxyalkynyls and mercaptoalkynyls; substituted cycloalkyls include moieties such as 4-chlorocyclohexyl; aryls include moieties such as napthyl; substituted aryls include moieties such as 3-bromo phenyl; aralkyls include moieties such as tolyl; heteroalkyls include moieties such as ethylthiophene; substituted heteroalkyls include moieties such as 3-methoxy-thiophene; alkoxy includes moieties such as methoxy; and phenoxy includes moieties such as 3-nitrophenoxy. Halo shall be understood to include fluoro, chloro, iodo and bromo.

The terms "effective amounts" and "sufficient amounts" for purposes of the present invention shall mean an amount which achieves a desired effect or therapeutic effect as such effect is understood by those of ordinary skill in the art.

### BRIEF DESCRIPTION OF DRAWINGS

FIG. 1 schematically illustrates a reaction scheme of preparing four-arm polyethylene glycol acids.
FIG. 2 schematically illustrates a reaction scheme of preparing 4arm-PEG-Gly-(7-ethyl-10-hydroxycamptothecin) described in Examples 3-7.
FIG. 3 schematically illustrates a reaction scheme of preparing 4arm-PEG-Ala-(7-ethyl-10-hydroxycamptothecin) described in Examples 8-12.
FIG. 4 schematically illustrates a reaction scheme of preparing 4arm-PEG-Met-(7-ethyl-10-hydroxycamptothecin) described in Examples 13-16.
FIG. 5 schematically illustrates a reaction scheme of preparing 4arm-PEG-Sar-(7-ethyl-10-hydroxycamptothecin) described in Examples 17-21.
FIG. 6 shows single dose and multiple dose efficacies in large MX-1 breast tumor xenografted mice described in Example 26.
FIG. 7 shows single dose efficacies in HT-29 colorectal tumor xenografted mice described in Example 27.
FIG. 8 shows multiple dose efficacies in HT-29 colorectal tumor xenografted mice described in Example 28.
FIG. 9 shows single dose efficacies in MiaPaCa-2 pancreatic tumor xenografted mice described in Example 29.
FIG. 10 shows multiple dose efficacies in MiaPaCa-2 pancreatic tumor xenografted mice described in Example 30.
FIG. 11 shows *in vitro* metabolism of 4arm-PEG-Gly-(7-ethyl-10-hydroxycamptothecin) as described in Example 31.
FIG. 12 shows stability of 4arm-PEG-Gly-(7-ethyl-10-hydroxycamptothecin) as described in Example 33.
FIG. 13 shows effect of pH on stability of 4arm-PEG-Gly-(7-ethyl-10-hydroxy-camptothecin) as described in Example 33.
FIG. 14 shows pharmacokinetic profiles of 4arm-PEG-Gly-(7-ethyl-10-hydroxy-camptothecin) as described in Example 34.

### DETAILED DESCRIPTION OF INVENTION

In one embodiment of the present invention, there are provided compounds of Formula (I): 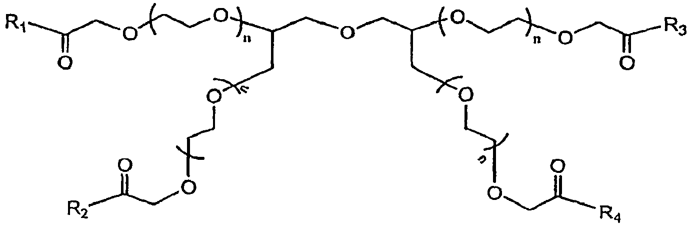 wherein
R₁, R₂, R₃ and R₄ are independently OH or (L)ₘ-D;
L is a bifunctional linker;
D is a residue of a camptothecin or a camptothecin analog, such as 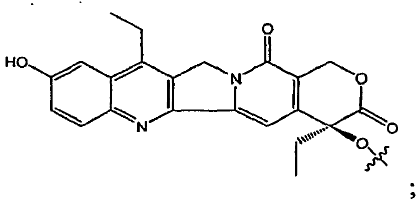
m is a positive integer from 1 to 10, and more preferably 1; and
n is a positive integer, preferably from 28 to 341, more preferably 227;
provided that R₁, R₂, R₃ and R₄ are not all OH.

### A. CAMPTOTHECIN AND RELATED CAMPTOTHECIN ANALOGS

Camptothecin is a water-insoluble cytotoxic alkaloid produced by *camptoteca accuminata* trees indigenous to China and *nothapodytes foetida* trees indigenous to India. Camptothecin and related compounds and analogs are also known to be potential anticancer or antitumor agents and have been shown to exhibit these activities *in vitro* and *in vivo* in laboratory animals.

Camptothecin and certain related analogues share the structure:

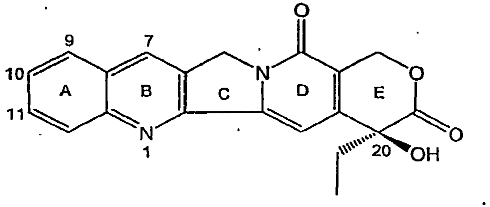

From this core structure, several known analogs have been prepared. For example, the A ring in either or both of the 10- and 11-positions can be substituted with an OH. The A ring can also be substituted with a straight or branched C₁₋₃₀ alkyl or C₁₋₁₇ alkoxy, optionally linked to the ring by a heteroatom i.e.- O or -S. The B ring can be substituted in the 7-position with a straight or branched C₁₋₃₀ alkyl (preferably C₂ alkyl), C₅₋₈ cycloakyl, C₁₋₃₀ alkoxy, phenyl alkyl, etc., alkyl carbamate, alkyl carbazides, phenyl hydrazine derivatives, etc. Other substitutions are possible in the C, D and E rings. See, for example, U.S. Patent Nos. 5,004,758; 4,943,579; RE 32,518. As the artisan will appreciate, the 10-hydroxy-camptothecin, 11-hydroxycamptothecin and the 10,11-dihydroxycamtothecin analogs occur naturally as one of the minor components in *C. Acuminata* and its relatives. Additional substitutions to these compounds, i.e. 7-alkyl-, 7-substituted alkyl-, 7-amino-, 7-aminoalkyl-, 7-aralkyl-, 9-alkyl-, 9-aralkyl- camptothecin etc. derviatives can be made using known synthetic techniques without undue experimentation. Some camptotheca alkaloids have the structure shown below:

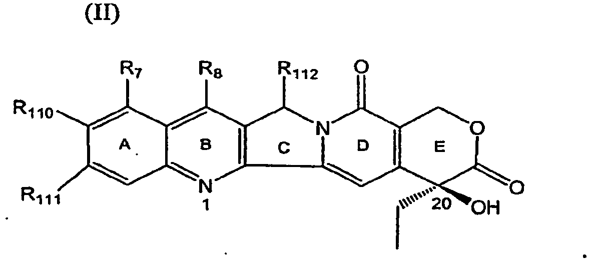

In the structure shown above, R₇ is one of NO₂, NH₂, N₃, hydrogen, halogen (F, Cl, Br, I), COOH, OH, O-C₁₋₈ alkyl, SH, S-C₁₋₃ alkyl, CN, CH₂NH₂, NH-C₁₋₃ alkyl, CH₂-NH-C₁₋₃ alkyl, N(C₁₋₃ alkyl)₂, CH₂N(C₁₋₃alkyl), O-, NH- and S-CH₂CH₂N(CH₂CH₂OH₂, O-, NH- and S-CH₂CH₂CH₂N(CH₂CH₂OH)₂, O-, NH- and S-CH₂CH₂N(CH₂CH₂CH₂OH)₂, O-, NH- and S-CH₂CH₂CH₂N(CH₂CH₂CH₂OH₂)₂, O-, NH- and S-CH₂CH₂N(C₁₋₃ alkyl)₂, O-, NH- and S-CH₂CH₂CH₂N(C₁₋₃ alkyl)₂, CHO or C₁₋₃ alkyl.

R₈ in the structure (II) shown above can be H or C₁₋₈ alkyl (preferably C₂ alkyl) or CH₂NR₉R₁₀ where
(a) R₉ and R₁₀ are, independently, hydrogen, C₁₋₆ alkyl, C₃₋₇ cycloalkyl, C₃₋₇ cycloalkyl-C₁₋₆ alkyl, C₂₋₆ alkenyl, hydroxy-C₁₋₆ alkyl, C₁₋₆ alkoxy-C₁₋₆ alkyl; alternatively
(b) R₉ can be hydrogen, C₁₋₆ alkyl, C₃₋₇ cycloalkyl, C₃₋₇cycloalkyl-C₁₋₆ alkyl, C₂₋₆ alkenyl, hydroxy-C₁₋₆ alkyl, C₁₋₆ alkoxy-C₁₋₆ alkyl and R₁₀ can be -COR₁₁ where R₁₁ is hydrogen, C₁₋₆ alkyl, perhalo-C₁₋₆ alkyl, C₃₋₇ cycloalkyl, C₃₋₇ cycloalkyl-C₁₋₆ alkyl, C₂₋₆ alkenyl, hydroxy-C₁₋₆ alkyl, C₁₋₆ alkoxy, C₁₋₆ alkoxy-C₁₋₆ alkyl; or
(c) R₉ and R₁₀ taken together with the nitrogen atom to which they are attached form a saturated 3-7 membered heterocyclic ring which may contain a O, S or NR₁₂ group, where R₁₂ is hydrogen, C₁₋₆ alkyl, perhalo-C₁₋₆ alkyl, aryl, aryl substituted with one or more groups selected from the group consisting of C₁₋₆ alkyl, halogen, nitro, amino, C₁₋₆ alkylamino, perhalo-C₁₋₆ alkyl, hydroxy-C₁₋₆ alkyl, C₁₋₆ alkoxy, C₁₋₆ alkoxy-C₁₋₆ alkyl and -COR₁₃ where R₁₃ is hydrogen, C₁₋₆ alkyl, perhalo-C₁₋₆ alkyl, C₁₋₆ alkoxy, aryl, and aryl substituted with one or more of C₁₋₆ alkyl, perhalo-C₁₋₆ alkyl, hydroxy-C₁₋₆ alkyl, or C₁₋₆ alkoxy-C₁₋₆ alkyl groups;
R₁₁₀-R₁₁₁ are each independently selected from the group consisting of hydrogen; halo; acyl; alkyl (e.g., C₁₋₆ alkyl); substituted alkyl; alkoxy (e.g., C₁₋₆ alkoxy); substituted alkoxy; alkenyl; alkynyl; cycloalkyl; hydroxyl; cyano; nitro; azido; amido; hydrazine; amino; substituted amino (e.g., monoalkylamino and dialkylamino); hydroxcarbonyl; alkoxycarbonyl; alkylcarbonyloxy; alkylcarbonylamino; carbamoyloxy; arylsulfonyloxy; alkylsulfonyloxy; -C(R₁₁₇)=N-(O)ⱼ-R₁₁₈ wherein R₁₁₇ is H, alkyl, alkenyl, cycloalkyl, or aryl, j is 0 or 1, and R₁₁₈ is H, alkyl, alkenyl, cycloalkyl, or heterocycle; and R₁₁₉C(O)O-wherein R₁₁₉ is halogen, amino, substituted amino, heterocycle, substituted heterocycle, or R₁₂₀-O-(CH₂)ₖ- where k is an integer of 1-10 and R₁₂₀ is alkyl, phenyl, substituted phenyl, cycloalkyl, substituted cycloalkyl, heterocycle, or substituted heterocycle; or
R₇ together with R₁₁₀ or R₁₁₀ together with R₁₁₁ form substituted or unsubstituted methylenedioxy, ethylenedioxy, or ethyleneoxy; and
R₁₁₂ is H or OR', wherein R' is alkyl, alkenyl, cycloalkyl, haloalkyl, or hydroxyalkyl.

Preferred aryl groups are phenyl and naphthyl. Suitable heterocyclic rings when R₉ and R₁₀ are taken together with the nitrogen atom to which they are attached include: aziridine, azetidine, pyrrolidine, piperidine, hexamethylenimine, imidazolidine, pyrazolidine, isoxazolidine, piperazine, N-methylpiperazine, tetrahydroazepine, N-methyl-tetrahydroazepine, thiazolidine, etc. After conjugation, the remaining camptothecin analog is referred to as the residue of the unconjugated compound.

For ease of description, the description refers to 7-ethyl-10-hydroxycamptothecin as the camptothecin analog as the preferred and illustrated compound. It will be understood that the claimed invention includes all such derivatives and analogs as defined in claim 1 so long as the analog has an OH such as the 20-OH group for the point of attachment to the polymer. The camptothecin or camptothecin analogs can be racemic mixtures or optically pure isomer. Preferably, a substantially pure and active form of such as the 20(S) camptothecin or camptothecin analog is employed in the multi-arm polymeric prodrugs.

### B. BIFUNCTIONAL LINKERS

In certain preferred aspects of the present invention, L is a residue of an amino acid. The amino acid which can be selected from any of the known naturally-occurring L-amino acids is, e.g., alanine, valine, leucine, isoleucine, glycine, serine, threonine, methionine, cysteine, phenylalanine, tyrosine, tryptophan, aspartic acid, glutamic acid, lysine, arginine, histidine, proline, and/or a combination thereof, to name but a few. In alternative aspects, L can be a peptide residue. The peptide can range in size, for instance, from about 2 to about 10 amino acid residues.

Derivatives and analogs of the naturally occurring amino acids, as well as various art-known non-naturally occurring amino acids (D or L), hydrophobic or non-hydrophobic, are also contemplated to be within the scope of the invention. The amino acid analogs and derivates are selected from: 2-aminoadipic acid, 3-aminoadipic acid, beta-alanine, beta-aminopropionic acid, 2-aminobutyric acid, 4-aminobutyric acid, piperidinic acid, 6-aminocaproic acid, 2-aminoheptanoic acid, 2-aminoisobutyric acid, 3-aminoisobutyric acid, 2-aminopimelic acid, 2,4-aminobutyric acid, desmosine, 2,2-diaminopimelic acid, 2,3-diaminopropionic acid, n-ethylglycine, N-ethylasparagine, 3-hydroxyproline, 4-hydroxyproline, isodesmosine, allo-isoleucine, N-methylglycine or sarcosine, N-methyl-isoleucine, 6-N-methyl-lysine, N-methylvaline, norvaline, norleucine, ornithine. Some preferred L groups include glycine, alanine, methionine or sarcosine residues. More preferably, compounds of the present invention include a glycine residue as the linker group (L).

In another aspect of the present invention, L after attachment between the camptothecin analog and polymer is selected among:

-[C(O)]ᵥNR₂₁(CR₂₂R₂₃O)ₜ- ,

-[C(O)]ᵥNR₂₁(CR₂₂R₂₃O)ₜ(CR₂₄R₂₅)_{y}O-,

-[C(O)]ᵥNR₂₁(CR₂₂R₂₃O)ₜ(CR₂₄R₂₅)_{y}-,

-[C(O)]ᵥNR₂₁(CR₂₂R₂₃)ₜO-,

-[C(O)]ᵥNR₂₁(CR₂₂R₂₃)ₜ(CR₂₄CR₂₅O)_{y}NR₂₆-,

-[C(O)]ᵥO(CR₂₂R₂₃)ₜNR₂₆- ,

-[C(O)]ᵥO(CR₂₂R₂₃)ₜO- ,

-[C(O)]ᵥNR₂₁(CR₂₂R₂₃)ₜNR₂₆- ,

-[C(O)]ᵥNR₂₁(CR₂₂R₂₃)ₜ(CR₂₄CR₂₅O)_{y}- ,

-[C(O)]ᵥNR₂₁(CR₂₂R₂₃)ₜ(CR₂₄CR₂₅)_{y}NR₂₆- ,

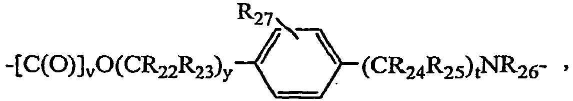

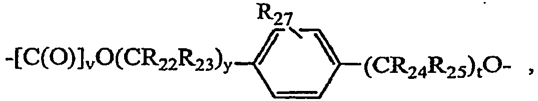

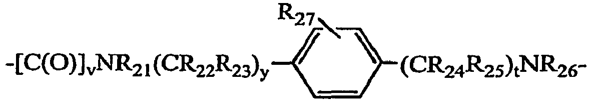

and 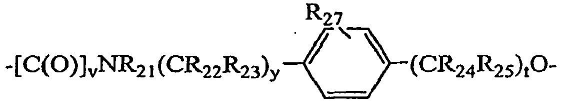 wherein:
R₂₁-R₂₆ are independently selected from the group consisting of hydrogen, C₁₋₆ alkyls, C₂₋₆ alkenyls, C₂₋₆ alkynyls, C₃₋₁₉ branched alkyls, C₃₋₈ cycloalkyls, C₁₋₆ substituted alkyls, C₂₋₆ substituted alkenyls, C₂₋₆ substituted alkynyls, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy and C₁₋₆ heteroalkoxy;
R₂₇ is selected from the group consisting of hydrogen, C₁₋₆ alkyls, C₂₋₆ alkenyls, C₂₋₆ alkynyls, C₃₋₁₉ branched alkyls, C₃₋₈ cycloalkyls, C₁₋₆ substituted alkyls, C₂₋₆ substituted alkenyls, C₂₋₆ substituted alkynyls, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy, C₁₋₆ heteroalkoxy, NO₂, haloalkyl and halogen;
t and y are individually selected positive integers from about 1 to about 4; and
v is 0 or 1.

In some preferred aspects of the present invention, the compounds include one unit of the bifunctional linker and thus m is 1.

Additional linkers are found in Table 1 of Greenwald et al. (Bioorganic & Medicinal Chemistry, 1998, 6:551-562).

### C. MULTI-ARM POLYMERS

Multi-arm PEG's are those described in NOF Corp. Drug Delivery System catalog, Ver. 8, April 2006. One particularly preferred multi-arm PEG has the structure: 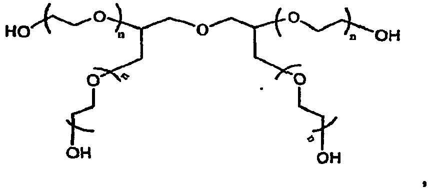 wherein n is a positive integer.

In one preferred embodiment of the invention, the degree of polymerization for the polymer (n) is from 28 to 341 to provide polymers having a total molecular weight of from about 5,000 Da to about 60,000 Da, and preferably from 114 to 227 to provide polymers having a total molecular weight of from 20,000 Da to 40,000 Da. This represents the number of repeating units in the polymer chain and is dependent on the molecular weight of the polymer. In one particularly preferred embodiment of the invention, n is 227.

### D. SYNTHESIS OF PRODRUGS

Generally, the prodrugs of the invention are prepared by reacting one or more equivalents of an activated multi-arm polymer with, for example, one or more equivalents per active site of camptothecin-amino acid conjugate under conditions which are sufficient to effectively cause the amino group to undergo a reaction with the carboxylic acid of the polymer and form a linkage.

More specifically, the methods can include:
1) providing one equivalent of a camptothecin or camptothecin analog containing an available 20-hydroxyl group and one or more equivalents of a bifunctinal linker containing an available carboxylic acid group;
2) reacting the two reactants to form a camptothecin-bifunctional linker intermediate in an inert solvent such as DCM (or DMF, chloroform, toluene or mixtures thereof) in the presence of a coupling reagent such as 1,(3-dimethyl aminopropyl) 3-ethyl carbodiimide (EDC), (or 1,3-diisopropylcarbodiimide (DIPC), any suitable dialkyl carbodiimide, Mukaiyama reagents, (e.g. 2-halo-1-alkyl-pyridinium halides) or propane phosphonic acid cyclic anhydride (PPACA), etc) and a suitable base such as DMAP; and
3) reacting one or more equivalents per active site (2 eq. in Example) of the resulting intermediate having an amine group and one equivalent of an activated polymer, such as a PEG-acid in an inert solvent such as DCM (or DMF, chloroform, toluene or mixtures thereof) in the presence of a coupling reagent such as 1,(3-dimethyl aminopropyl) 3-ethyl carbodiimide (EDC), PPAC (or 1,3-diisopropylcarbodiimide (DIPC), any suitable dialkyl carbodiimide, Mukaiyama reagents, (e.g. 2-halo-1-alkyl-pyridinium halides) or propane phosphonic acid cyclic anhydride (PPACA), etc.), and a suitable base such as DMAP, which are available, for example, from commercial sources such as Sigma Chemical, or synthesized using known techniques, at a temperature from 0°C up to 22°C.

In one preferred aspect, the 10-hydroyl group of 7-ethyl-10-hydroxycamptothecin is protected prior to step 1).

Aromatic protecting groups for the OH group of camptothecin or camptothecin analog such as 10-hydroxyl group in 7-ethyl-10-hydroxycamptothecin are preferred because 7-ethyl-10-hydroxycamptothecin intermediates thereof have better solubility and can be purified in highly pure form efficiently and effectively. For example, silyl-containing protecting groups such as TBDPSCl, TBDMSCl and TMSCl can be used to protect the 10-hydroxyl group in camptothecin or camptothecin analog.

The activated polymer, i.e., a polymer containing 1-4 terminal carboxyl acid groups can be prepared, for example, by converting NOF Sunbright-type or other branched polymers having terminal OH groups into the corresponding carboxyl acid derivatives using standard techniques well known to those of ordinary skill. See, for example, Examples 1-2 herein as well as commonly assigned U.S. Patent No. 5,605,976.

The first and second coupling agents can be the same or different.

Examples of preferred bifunctional linker groups include glycine, alanine, methionine, sarcosine, etc. and syntheses are shown in the Examples. Alternative and specific syntheses are provided in the examples.

For example, the compounds of the present invention are among: 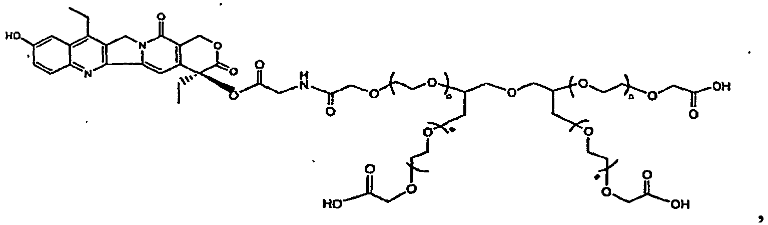 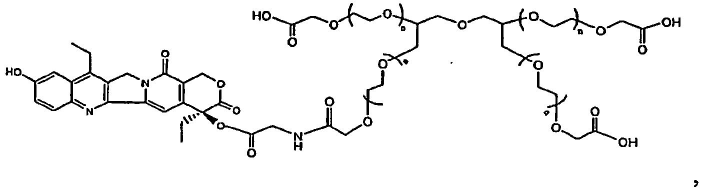 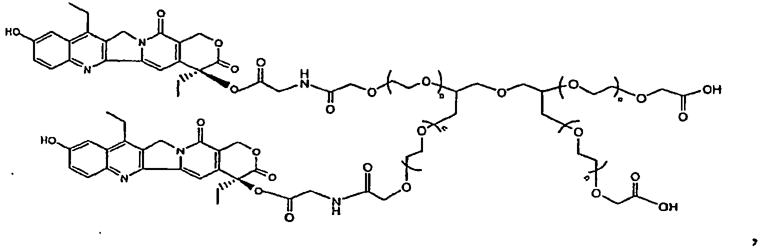 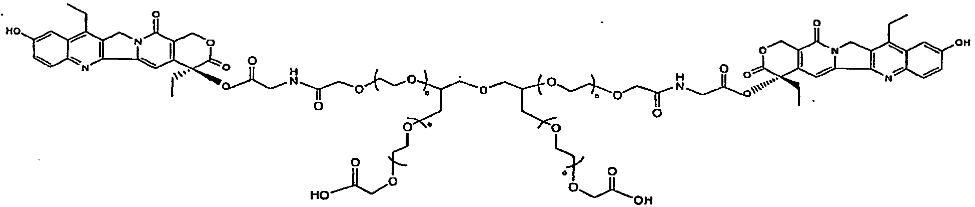 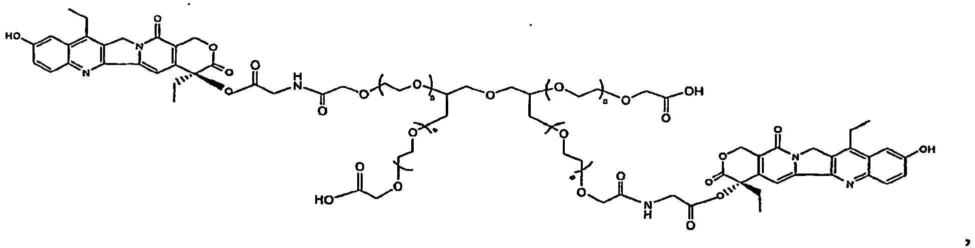 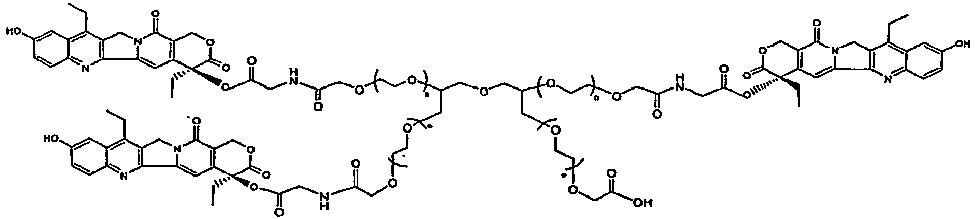 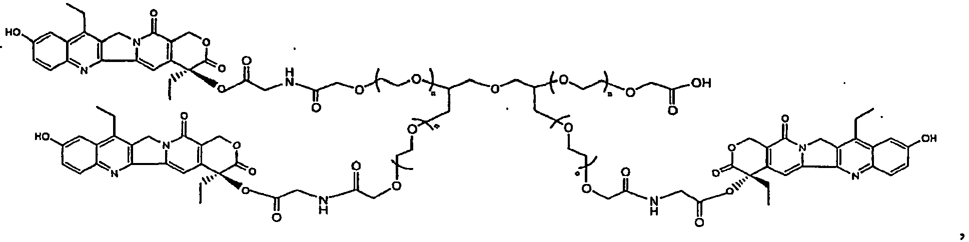 and

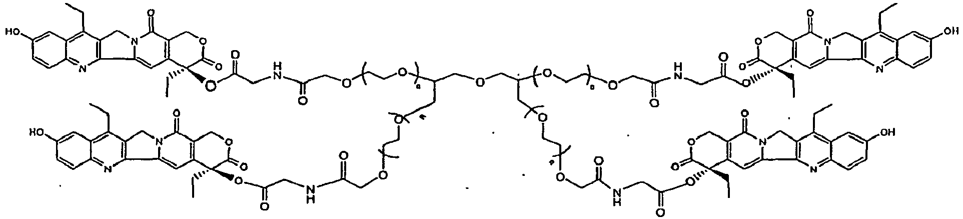

One particularly preferred compound is 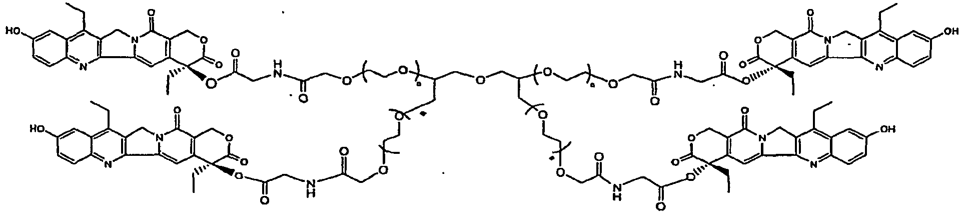 wherein all four arms of the polymer are conjugated to 7-ethyl-10-hydroxycamptothecin through glycine. HPLC analysis of compounds made in accordance with this aspect of the inventions shows that on average, four 7-ethyl-10-hydroxycamptothecin molecules are conjugated to one PEG molecule (4% by weight).

### E. COMPOSITIONS/FORMULATIONS

Pharmaceutical compositions containing the polymer conjugates of the present invention may be manufactured by processes well known in the art, e.g., using a variety of well-known mixing, dissolving, granulating, levigating, emulsifying, encapsulating, entrapping or lyophilizing processes. The compositions may be formulated in conjunction with one or more physiologically acceptable carriers comprising excipients and auxiliaries which facilitate processing of the active compounds into preparations which can be used pharmaceutically. Proper formulation is dependent upon the route of administration chosen. Parenteral routes are preferred in many aspects of the invention.

For injection, including, intravenous, intramusclular and subcutaneous injection, the compounds of the invention may be formulated in aqueous solutions, preferably in physiologically compatible buffers such as physiological saline buffer or polar solvents including, without limitation, a pyrrolidone or dimethylsulfoxide.

The compounds may also be formulated for parenteral administration, e.g., by bolus injection or continuous infusion. Formulations for injection may be presented in unit dosage form, e.g., in ampoules or in multi-dose containers. Useful compositions include, suspensions, solutions or emulsions in oily or aqueous vehicles, and may contain adjuncts such as suspending, stabilizing and/or dispersing agents. Pharmaceutical compositions for parenteral administration include aqueous solutions of a water soluble form, such as, a salt (preferred) of the active compound. Additionally, suspensions of the active compounds may be prepared in a lipophilic vehicle. Suitable lipophilic vehicles include fatty oils such as sesame oil, synthetic fatty acid esters such as ethyl oleate and triglycerides, or materials such as liposomes. Aqueous injection suspensions may contain substances that increase the viscosity of the suspension, such as sodium carboxymethyl cellulose, sorbitol, or dextran. Optionally, the suspension may also contain suitable stabilizers and/or agents that increase the solubility of the compounds to allow for the preparation of highly concentrated solutions. Alternatively, the active ingredient may be in powder form for constitution with a suitable vehicle, e.g., sterile, pyrogen-free water, before use.

For oral administration, the compounds can be formulated by combining the active compounds with pharmaceutically acceptable carriers well-known in the art. Such carriers enable the compounds of the invention to be formulated as tablets, pills, lozenges, dragees, capsules, liquids, gels, syrups, pastes, slurries, solutions, suspensions, concentrated solutions and suspensions for diluting in the drinking water of a patient, premixes for dilution in the feed of a patient, and the like, for oral ingestion by a patient. Pharmaceutical preparations for oral use can be made using a solid excipient, optionally grinding the resulting mixture, and processing the mixture of granules, after adding other suitable auxiliaries if desired, to obtain tablets or dragee cores. Useful excipients are, in particular, fillers such as sugars, including lactose, sucrose, mannitol, or sorbitol, cellulose preparations such as, for example, maize starch, wheat starch, rice starch and potato starch and other materials such as gelatin, gum tragacanth, methyl cellulose, hydroxypropyl- methylcellulose, sodium carboxy- methylcellulose, and/or polyvinylpyrrolidone (PVP). If desired, disintegrating agents may be added, such as cross-linked polyvinyl pyrrolidone, agar, or alginic acid. A salt such as sodium alginate may also be used.

For administration by inhalation, the compounds of the present invention can conveniently be delivered in the form of an aerosol spray using a pressurized pack or a nebulizer and a suitable propellant.

The compounds may also be formulated in rectal compositions such as suppositories or retention enemas, using, e.g., conventional suppository bases such as cocoa butter or other glycerides.

In addition to the formulations described previously, the compounds may also be formulated as depot preparations. Such long acting formulations may be administered by implantation (for example, subcutaneously or intramuscularly) or by intramuscular injection. A compound of this invention may be formulated for this route of administration with suitable polymeric or hydrophobic materials (for instance, in an emulsion with a pharmacologically acceptable oil), with ion exchange resins, or as a sparingly soluble derivative such as, a sparingly soluble salt.

Other delivery systems such as liposomes and emulsions can also be used.

Additionally, the compounds may be delivered using a sustained-release system, such as semi-permeable matrices of solid hydrophobic polymers containing the therapeutic agent. Various sustained-release materials have been established and are well known by those skilled in the art. Sustained-release capsules may, depending on their chemical nature, release the compounds for a few weeks up to over 100 days. Depending on the chemical nature and the biological stability of the particular compound, additional stabilization strategies may be employed.

### F. DOSAGES

A therapeutically effective amount refers to an amount of compound effective to prevent, alleviate or ameliorate the camptothecin or related analog, i.e., 7-ethyl-10-hydroxycamptothecin -susceptible condition. Determination of a therapeutically effective amount is well within the capability of those skilled in the art, especially in light of the disclosure herein.

For any compound used in the methods of the invention, the therapeutically effective amount can be estimated initially from *in vitro* assays. Then, the dosage can be formulated for use in animal models so as to achieve a circulating concentration range that includes the effective dosage. Such information can then be used to more accurately determine dosages useful in patients.

Toxicity and therapeutic efficacy of the compounds described herein can be determined by standard pharmaceutical procedures in cell cultures or experimental animals using methods well-known in the art. The dosage, of course, can vary depending upon the dosage form and route of administration. The exact formulation, route of administration and dosage can be selected by the individual physician in view of the patient's condition. In general, however, the presently preferred dosage range for systemic delivery of a compound of this invention will be from 1 to 100 mg/kg/week and is preferably from 2 to 60 mg/kg/week.

The compositions may be administered once daily or divided into multiple doses which can be given as part of a multi-week treatment protocol. The precise dose will depend on the stage and severity of the condition, the susceptibility of the tumor to the polymer-prodrug composition, and the individual characteristics of the patient being treated, as will be appreciated by one of ordinary skill in the art.

### G. METHODS OF TREATMENT

In view of the above, there are also provided medicament for treating a mammal, comprising administering an effective amount of a compound of the present invention of Formula (I), wherein D is a biologically active moiety, i.e., camptothecin analog according to claim 1 to a patient in need thereof. In one preferred aspect of the invention, D is a residue of 7-ethyl-10-hydroxycamptothecin.

Another aspect of the present invention provides compositions for the treatment for various medical conditions in mammals. The compositions are useful for, among other things, treating neoplastic disease, reducing tumor burden, preventing metastasis of neoplasms and preventing recurrences of tumor/neoplastic growths in mammals. In alternative aspects, the cancer being treated can be one or more of the following: solid tumors, lymphomas, small cell lung cancer, acute lymphocytic leukemia (ALL), pancreatic cancer, glioblastoma, ovarian cancer, gastric cancers.

The amount of the composition, e.g., used as a prodrug, that is administered will depend upon the parent molecule included therein. Generally, the amount ofprodrug used in the treatment is that amount which effectively achieves the desired therapeutic result in mammals. Naturally, the dosages of the various prodrug compounds will vary somewhat depending upon the parent compound, rate of *in vi vo* hydrolysis, molecular weight of the polymer, etc. In general, polymeric ester derivatives of camptothecin and related compositions are administered in amounts ranging from 1 to 100 mg/kg/week and preferably from 2 to 60 mg/kg/week. The range set forth above is illustrative and those skilled in the art will determine the optimal dosing of the prodrug selected based on clinical experience and the treatment indication.

In alternative aspects of the invention, the treatment includes administering an effective amount of the compounds described herein to a mammal or patient in need thereof suffering from conditions which can be treated by topoisomerase interference.

### EXAMPLES

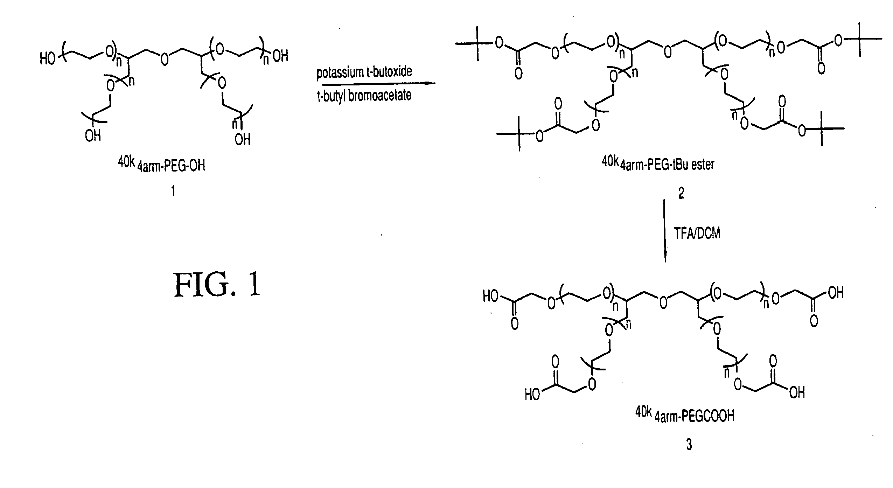

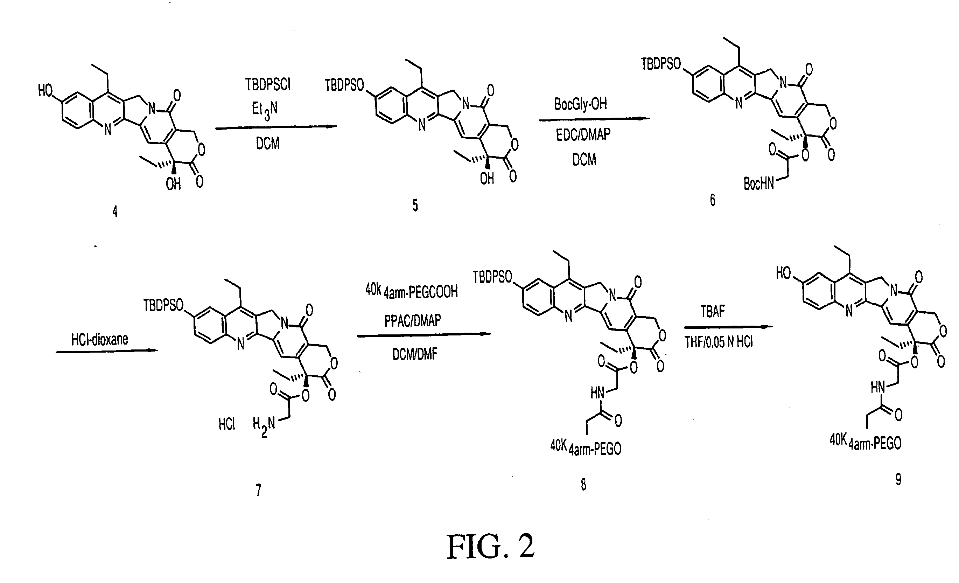

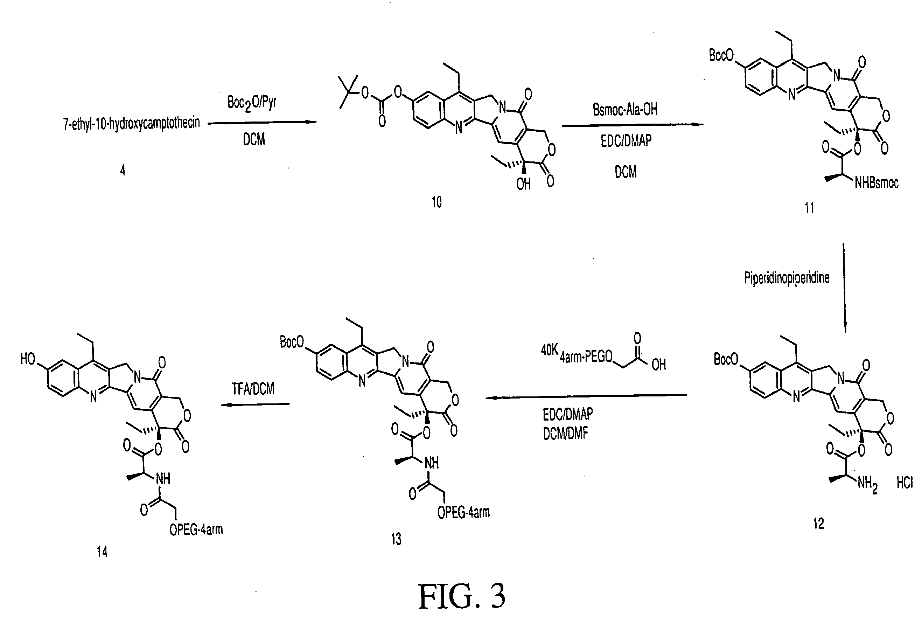

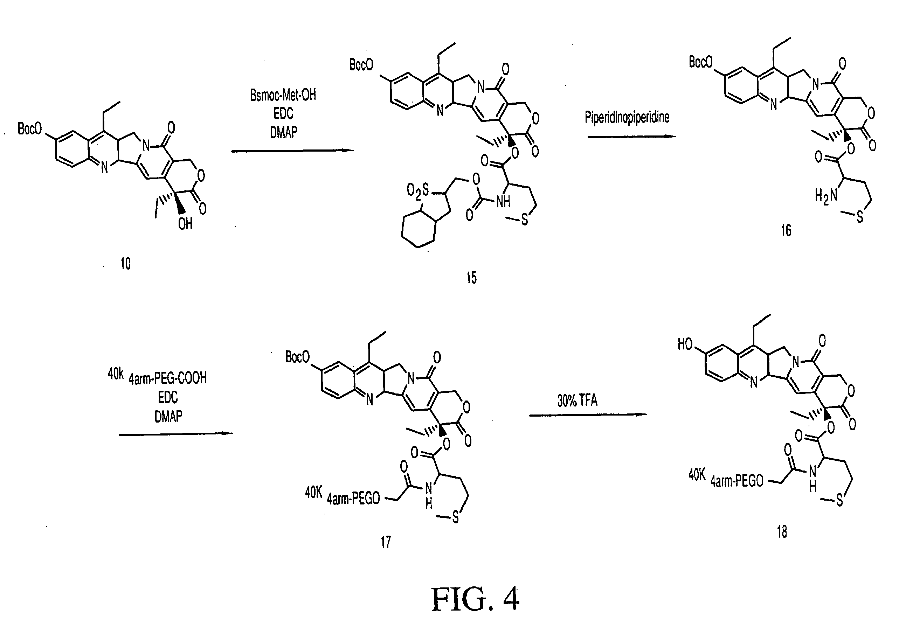

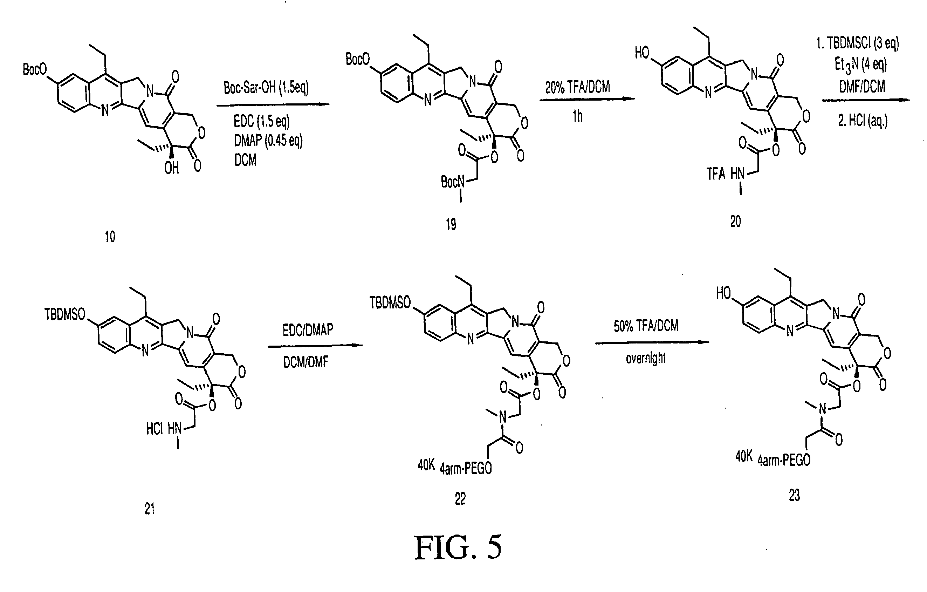

The following examples serve to provide further appreciation of the invention. The underlined and bold-faced numbers recited in the Examples correspond to those shown in the FIGS. 1-5.
**General Procedures.** All reactions were run under an atmosphere of dry nitrogen or argon. Commercial reagents were used without further purification. All PEG compounds were dried under vacuum or by azeotropic distillation from toluene prior to use. ¹³C NMR spectra were obtained at 75.46 MHz using a Varian Mercury^{®}300 NMR spectrometer and deuterated chloroform and methanol as the solvents unless otherwise specified. Chemical shifts (δ) are reported in parts per million (ppm) downfield from tetramethylsilane (TMS).
**HPLC Method.** The reaction mixtures and the purity of intermediates and final products were monitored by a Beckman Coulter System Gold^{®} HPLC instrument. It employs a ZOBAX^{®} 300SB C8 reversed phase column (150 x 4.6 mm) or a Phenomenex Jupiter^{®} 300A C18 reversed phase column (150 x 4.6 mm) with a multiwavelength UV detector, using a gradient of 10-90 % of acetonitrile in 0.05 % trifluoroacetic acid (TFA) at a flow rate of 1 mL/min.)

### EXAMPLE 1. ^{40k} 4arm-PEG-tBu ester (compound 2):

^{40k} 4arm-PEG-OH (12.5 g, 1 eq.) was azeotroped with 220 mL of toluene to remove 35 mL of toluene/water. The solution was cooled to 30 ° C and 1.0 M potassium t-butoxide in t-butanol (3.75 mL, 3eq x 4 =12 eq.) was added. The mixture was stirred at 30 ° C for 30 min and then t-butyl bromoacetate (0.975 g, 4 eq. x 4 = 16 eq.) was added. The reaction was kept at 30 ° C for 1 hour and then was cooled to 25 °C. 150 mL of ether was slowly added to precipitate product. The resulting suspension was cooled to 17 °C and stayed at 17 °C for half hour. The crude product was filtered and the wet cake was washed with ether twice (2 x 125 mL). The isolated wet cake was dissolved in 50 ml of DCM and the product was precipitated with 350 ml of ether and filtered. The wet cake was washed with ether twice (2 x 125 mL). The product was dried under vacuum at 40°C (yield = 98%, 12.25 g). ¹³C NMR (75.4 MHz, CDCl₃): δ 27.71, 68.48-70.71 (PEG), 80.94, 168.97.

### EXAMPLE 2. ^{40k} 4arm-PEG acid (compound 3):

^{40k} 4arm-PEG-tBu ester (compound **2**, 12 g) was dissolved in 120 mL of DCM and then 60 mL of TFA were added. The mixture was stirred at room temperature for 3 hours and then the solvent was removed under vacuum at 35 °C. The resulting oil residue was dissolved in 37.5 mL of DCM. The crude product was precipitated with 375 mL of ether. The wet cake was dissolved in 30 mL of 0.5% NaHCO₃. The product was extracted with DCM twice (2 x150ml). The combined organic layers were dried over 2.5 g of MgSO₄. The solvent was removed under vacuum at room temperature. The resulting residue was dissolved in 37.5 mL of DCM and the product was precipitated with 300 mL of ether and filtered. The wet cake was washed with ether twice (2 x 125ml). The product was dried under vacuum at 40 °C (yield = 90%, 10.75 g). ¹³C NMR (75.4 MHz, CDCl₃): δ 67.93-71.6 (PEG), 170.83.

### EXAMPLE 3. TBDPS-(10)-(7-ethyl-10-hydroxycamptothecin) (compound 5):

To a suspension of 7-ethyl-10-hydroxycamptothecin (compound **4**, 2.0 g, 5.10 mmol, 1 eq.) in 100 mL of anhydrous DCM were added Et₃N (4.3 mL, 30.58 mmol, 6 eq.) and TBDPSCl (7.8 mL, 30.58 mmol, 6 eq.). The reaction mixture was heated to reflux overnight and then, was washed with a 0.2 N HCl solution (2 x 50 mL), a saturated NaHCO₃ solution (100 mL) and brine (100 mL). The organic layer was dried over MgSO₄, filtered and evaporated under vacuum. The residue was dissolved in anhydrous DCM and precipitated by addition of hexanes. The precipitation with DCM/hexanes was repeated to get rid of excess TBDPSCl. The solids were filtered and dried under vacuum to give 2.09 g of product. (65% yield). ¹H NMR (300 MHz, CDCl₃): δ 0.90 (3 H, t, J = 7.6 Hz), 1.01 (3 H, t, J = 7.3 Hz), 1.17 (9H, s), 1.83-1.92 (2H, m), 2.64 (2H, q, 6.9 Hz), 3.89 (1 H, s, OH), 5.11 (2H, s), 5.27 (1H, d, J = 16.1 Hz), 5.72 (1H, d, J = 16.4 Hz), 7.07 (2 H, d, J = 2.63 Hz), 7.36-7.49 (7 H, m), 7.58 (1 H, s), 7.75-7.79 (4H, m), 8.05 (1 H, d, J = 9.4 Hz). ¹³C NMR (75.4 MHz, CDCl₃): δ 7.82, 13.28, 19.52, 22.86, 26.48, 31.52, 49.23, 66.25, 72.69, 97.25, 110.09, 117.57, 125.67, 126.57, 127.65, 127.81, 130.02, 131.69, 131.97, 135.26, 143.51, 145.05, 147.12, 149.55, 149.92, 154.73, 157.43, 173.72.

### EXAMPLE 4. TBDPS-(10)-(7-ethyl-10-hydroxycamptothecin)-(20)-Gly-Boc (compound 6):

To a 0 °C solution of TBDPS-(10)-(7-ethyl-10-hydroxycamptothecin) (compound 5, 3.78 g, 5.99 mmol, 1 eq.) and Boc-Gly-OH (1.57 g, 8,99 mmol, 1.5 eq.) in 100 mL of anhydrous DCM was added EDC (1.72 g, 8.99 mmol, 1.5 eq.) and DMAP (329 mg, 2.69 mmol, 0.45 eq.). The reaction mixture was stirred at 0 °C until HPLC showed complete disappearance of the starting material (approx. 1 hour and 45 minutes). The organic layer was washed with a 0.5% NaHCO₃ solution (2 x 50 mL), water (1 x 50 mL), a 0.1 N HCl solution (2 x 50 mL) and brine (1 x 50 mL); and dried over MgSO₄. After filtration and evaporation under vacuum, 4.94 g of crude product were obtained (quantitative yield). The crude solid was used in the next reaction without further purification. ¹H NMR (300 MHz, CDCl₃): δ 0.89 (3 H, t, J = 7.6 Hz), 0.96 (3 H, t, J = 7.5 Hz), 1.18 (9H, s), 1.40 (9H, s), 2.07-2.29 (3H, m), 2.64 (2H, q, 7.5 Hz), 4.01-4.22 (2H, m), 5.00 (1 H, br s), 5.01 (2H, s), 5.37 (1H, d, J = 17.0 Hz), 5.66 (1H, d, J = 17.0 Hz), 7.08 (1 H, d, J = 2.34 Hz), 7.16 (1H, s), 7.37-7.50 (7 H, m), 7.77 (4H, d, J = 7.6 Hz), 8.05 (1 H, d, J = 9.4 Hz). ¹³C NMR (75.4 MHz, CDCl₃): δ 7.52,13.30, 19.50, 22.86, 26.45, 28.21, 31.64, 42.28, 49.14, 67.00, 76.65, 79.96, 95.31, 110.13, 118.98, 125.75, 126.45, 127.68, 127.81, 130.03, 131.54, 131.92, 135.25, 143.65, 144.91, 145.19, 147.08, 149.27, 154.75, 155.14, 157.10, 166.98, 169.17.

### EXAMPLE 5. TBDPS-(10)-(7-ethyl-10-hydroxycamptothecin)-(20)-Gly•HCl (compound 7):

To a solution of TBDPS-(10)-(7-ethyl-10-hydroxycamptothecin)-(20)-Gly-Boc (compound 6, 1 g, 1.27 mmol) in 5 mL anhydrous dioxane was added 5 mL of a 4 M solution of HCl in dioxane. The reaction mixture was stirred at room temperature until HPLC showed complete disappearance of the starting material (1 hour). The reaction mixture was added to 50 mL of ethyl ether and the resulting solid was filtered. The solid was dissolved in 50 mL DCM and washed with brine (pH was adjusted to 2.5 by addition of a saturated NaHCO₃ solution). The organic layer was dried over MgSO₄, filtered and evaporated under vacuum. The residue was dissolved in 5 mL of DCM and precipitated by addition of 50 mL ethyl ether. Filtration afforded 770 mg (84 % yield) final product. ¹H NMR (300 MHz, CDCl₃): δ 0.84 (3 H, t, J = 7.6 Hz), 1.05 (3 H, t, J = 7.3 Hz), 1.16 (9H, s), 2.15-2.30 (3H, m), 2:59 (2H, q, 7.6 Hz), 4.16 (1H, d, J = 17.9 Hz), 4.26 (1H, d, J = 17.9 Hz), 5.13 (2H, s), 5.46 (1H, d, J = 17.0 Hz), 5.60 (1H, d, J = 17.0 Hz), 7.11 (1 H, d, J = 2.34 Hz), 7.30 (1H, s), 7.40-7.51 (6 H, m), 7.56 (1H, dd, J = 2.34, 9.4 Hz), 7.77 (4H, dd, J = 7.6, 1.6 Hz), 7.98 (1 H, d, J = 9.1 Hz). ¹³C NMR (75.4 MHz, CDCl₃): δ 8.09, 13.72, 20.26, 23.61, 26.94, 31.83, 41.01, 50.71, 67.62, 79.51, 97.03, 111.65, 119.69, 127.13, 128.97, 128.99, 129.11, 131.43, 131.96, 133.00, 133.03,136.51, 145.62, 145.81, 147.24, 148.29, 150.58, 156.27, 158.68, 167.81, 168.34.

### EXAMPLE 6. ^{40k} 4arm-PEG-Gly-(20)-(7-ethyl-10-hydroxycamptothecin)-(10)-TBDPS (compound 8):

To a solution of ^{40k} 4arm-PEGCOOH (compound 3, 1.4 g, 0.036 mmol, 1 eq.) in 14 mL of anhydrous DCM was added TBDPS-(10)-(7-ethyl-10-hydroxycamptothecin)-(20)-Gly•HCl (compound 7, 207 mg, 0.29 mmol, 2.0 eq. per active site), DMAP (175 mg, 1.44 mmol, 10 eq.) and PPAC (0.85 mL of a 50% solution in EtOAc, 1.44 mmol, 10 eq.). The reaction mixture was stirred at room temperature overnight and then, evaporated under vacuum. The resulting residue was dissolved in DCM and the product was precipitated with ether and filtered. The residue was recrystallized with DMF/IPA to give the product (1.25 g). ¹³C NMR (75.4 MHz, CDCl₃): δ 7.45, 13.20, 19.39, 22.73, 26.42, 31.67, 40.21, 49.01, 66.83, 95.16, 110.02, 118.83, 125.58, 126.40, 127.53, 127.73, 129.96, 131.49, 131.76, 131.82, 135.12, 143.51, 144.78, 145.13, 146.95, 149.21, 154.61, 156.92, 166.70, 168.46, 170.30.

### EXAMPLE 7. ^{40k} 4arm-PEG-Gly(20)-(7-ethyl-10-hydroxycamptothecin) (compound 9):

To compound ^{40k} 4arm-PEG-Gly-(20)-(7-ethyl-10-hydroxycamptothecin)-(10)-TBDPS (compound 8, 1.25 g) was added a solution of TBAF (122 mg, 0.46 mmol, 4 eq.) in a 1:1 mixture of THF and a 0.05 M HCl solution (12.5 mL). The reaction mixture was stirred at room temperature for 4 hours and then, extracted with DCM twice. The combined organic phases were dried over MgSO₄, filtered and evaporated under vacuum. The residue was dissolved in 7 mL of DMF and precipitated with 37 mL IPA. The solid was filtered and washed with IPA. The precipitation with DMF/IPA was repeated. Finally the residue was dissolved in 2.5 mL of DCM and precipitated by addition of 25 mL of ether. The solid was filtered and dried at 40 °C in vacuum oven overnight (860 mg). ¹³C NMR (75.4 MHz, CDCl₃): δ 7.48, 13.52, 22.91, 31.67, 40.22, 49.12, 66.95, 94.82, 105.03, 118.68, 122.54, 126.37, 128.20, 131.36, 142.92, 144.20, 144.98, 147.25, 148.29, 156.44, 156.98, 166.82, 168.49, 170.39. This NMR data shows no sign of PEG-COOH which indicates that all of the COOH reacted. The loading, as determined by fluorescence detection was found to be 3.9 which is consistent with full loading of the 7-ethyl-10-hydroxycamptothecin on each of the four branches of the polymer. Repeated runs of this experiments at much larger scale yielded consistent results.

### EXAMPLE 8. Boc-(10)-(7-ethyl-10-hydroxycamptothecin) (compound 10):

To a suspension of 7-ethyl-10-hydroxycamptothecin (compound **4,** 2.45 g, 1 eq.) in 250 mL of anhydrous DCM at room temperature under N₂ were added di-tert-butyl dicarbonate (1.764 g, 1.3 eq.) and anhydrous pyridine (15.2 mL, 30 eq.). The suspension was stirred overnight at room temperature. The hazy solution was filtered through celite (10 g) and the filtrate was washed with 0.5 N HCl three times (3 x 150 mL) and a NaHCO₃ saturated solution (1 x 150ml). The solution was dried over MgSO₄ (1.25 g). The solvent was removed under vacuum at 30°C. The product was dried under vacuum at 40°C (yield = 82%, 2.525g) ¹³C NMR (75.4 MHz, CDCl₃) d 173.53, 157.38, 151.60, 151.28, 150.02, 149.70, 147.00, 146.50, 145.15, 131.83, 127.19, 127.13, 124.98, 118.53, 113.88, 98.06, 84.26, 72.80, 66.18, 49.33, 31.62, 27.73, 23.17, 13.98, 7.90.

### EXAMPLE 9. Boc-(10)-(7-ethyl-10-hydroxycamptothecin)-(20)-Ala-Bsmoc (compound 11):

To a solution of Boc-(10)-(7-ethyl-10-hydroxycamptothecin) (compound **10,** 0.85g, 1.71 mmol) and Bsmoc-Ala (0.68g, 2.30 mmol) in anhydrous CH₂Cl₂ (20 mL) were added EDC (0.51g, 2.67 mmol) and DMAP (0.065g, 0.53 mmol) at 0°C. The mixture was stirred at 0°C for 45 min under N₂, then warmed up to room temperature. When completion of the reaction was confirmed by HPLC, the reaction mixture was washed with 1% NaHCO₃ (2 x 50 ml), H₂O (50 mL) and 0.1 N HCl (2 x 50 mL). The organic phase was dried with anhydrous MgSO₄ and filtrated. Solvent was removed under reduced pressure. The resulting solid was dried under vacuum below 40 ° C overnight to give the product of 1.28 g with the yield of 95%. ¹³C NMR (75.4 MHz, CDCl₃) d : 171.16,166.83, 157.16, 154.78, 151.59, 151.33, 149.82, 147.17, 146.68, 145.35, 145.15, 139.08, 136.88, 133.60, 131.83, 130.45, 130.40, 130.33, 127.40, 127.08, 125.32, 125.14, 121.38, 120.01, 114.17, 95.90, 84.38, 77.19, 76.64, 67.10, 56.66, 53.45, 49.96, 49.34, 31.7, 27.76, 17.94, 14.02, 7.53. ESI-MS, 786.20 [M + H]⁺.

### EXAMPLE 10. Boc-(10)-(7-ethyl-10-hydroxycamptothecin)-(20)-Ala (compound 12):

A solution of Boc-(10)-(7-ethyl-10-hydroxycamptothecin)-(20)-Ala-Bsmoc (compound **11,** 4.2 g, 5.35 mmol) and 4-piperidinopiperidine (1.17g, 6.96 mmol) in anhydrous CH₂Cl₂ (200 ml) was stirred at room temperature for 5 hours. This mixture was then washed with 0.1 N HCl (2 x 40ml), followed by drying the organic layer over anhydrous MgSO₄. This solution was filtered, and the solvent was removed by vacuum distillation to yield 2.8 g of product with purity of 93%, determined by HPLC. This product was further purified by trituration with ether (3 X 20 ml), and then trituration with ethyl acetate (4 x 20ml) to yield 1.52 g (2.70 mmol) with purity 97%. ¹³C NMR (75.4 MHz, CDCl₃) d 168.39, 166.63, 156.98, 151.20, 151.15, 149.69, 146.67, 146.56, 145.37, 144.53, 131.66, 127.13, 124.99, 119.80, 113.82, 96.15, 84.21, 77.67, 67.16, 49.48, 49.06, 31.56, 27.74, 23.14, 15.98, 13.98, 7.57.

### EXAMPLE 11. ^{40k} 4arm-PEG-Ala-(20)-(7-ethyl-10-hydroxycamptothecin)-(10)-Boc (compound 13):

To anhydrous CH₂Cl₂ (100 mL) Boc-(10)-(7-ethyl-10-hydroxycamptothecin)-(20)-Ala (compound **12,** 1.50 g, 2.5 mmol) and 4armPEG-COOH (compound 3, 10.01 g, 1.0 mmol) were added at room temperature. The solution was cooled to 0°C, followed by addition of EDC (0.29g, 1.5 mmol) and DMAP (0.30g, 2.5 mmol). The mixture was stirred at 0 °C for 1 hour under N₂. Then it was kept at room temperature overnight. The solvent was evaporated under reduced pressure. The residue was dissolved in 40 mL of DCM, and the crude product was precipitated with ether (300 mL). The wet solid resulting from filtration was dissolved in a mixture of DMF/IPA (60/240 mL) at 65°C. The solution was allowed to cool down to room temperature within 2 ∼ 3 hours, and the product was precipitated. Then, the solid was filtered and washed with ether (2 x 200 mL). The wet cake was dried under vacuum below 40°C overnight to give product of 8.5g.

### EXAMPLE 12. ^{40k} 4arm-PEG-Ala-(20)-(7-ethyl-10-hydroxycamptothecin) (compound 14):

To a solution (130 mL) of 30% TFA in anhydrous CH₂Cl₂ ^{40k} 4arm-PEG-Ala-(20)-(7-ethyl-10-hydroxycamptothecin)-(10)-Boc (compound 13, 7.98 g) was added at room temperature. The mixture was stirred for 3 hours, or until the disappearance of starting material was confirmed by HPLC. The solvents were removed as much as possible under vacuum at 35°C. The residues were dissolved in 50mL of DCM, and the crude product was precipitated with ether (350 mL) and filtered. The wet solid was dissolved in a mixture of DMF/IPA (50/200 mL) at 65°C. The solution was allowed to cool down to room temperature within 2 ∼ 3 hours, and the product was precipitated. Then the solid was filtered and washed with ether (2 x 200 mL). The wet cake was dried under vacuum below 40°C overnight to give product of 6.7g. ¹³C NMR (75.4 MHz, CDCl₃) d : 170.75, 169.30, 166.65, 157.00, 156.31, 148.36, 147.19, 145.03, 144.29, 143.00, 131.49, 128.26, 126.42, 122.47, 118.79, 105.10, 94.57, 78.08, 77.81, 77.20, 71.15, 70.88, 70.71, 70.33, 70.28, 70.06, 69.93, 69.57, 66.90, 49.14, 47.14, 31.53, 22.95, 17.78, 13.52, 7.46.

### EXAMPLE 13. Boc-(10)-(7-ethyl-10-hydroxycamptothecin)-(20)-Met-Bsmoc (compound 15):

To a solution of Boc-(10)-7-ethyl-10-hydroxycamptothecin (compound 10, 2.73g, 5.53 mmol) and Bsmoc-Met (3.19 g, 8.59 mmol) in anhydrous CH₂Cl₂ (50 mL) were added EDC (1.64g, 8.59 mmol) and DMAP (0.21g, 1.72 mmol) at 0°C. The mixture was stirred at 0°C for 45 minutes under N₂, then warmed up to room temperature. When completion of the reaction was confirmed by HPLC, the reaction mixture was washed with 1% NaHCO₃ (2 x 100 ml), H₂O (100 mL) and 0.1 N HCl (2 x 100 mL). The organic phase was dried with anhydrous MgSO₄ and filtrated. Solvents were removed under reduced pressure. The resulting solid was dried under vacuum below 40°C overnight to give the product of 4.2 g with the yield of 88 %. ¹³C NMR (75.4 MHz, CDCl₃) d: 170.3, 166.8, 157.1, 155.2, 151.4, 151.2, 149.7, 147.0, 146.6, 145.3, 145.1, 138.9, 136.6, 133.5, 131.7, 130.5, 130.3, 130.2, 127.3, 127.0, 125.3, 125.1, 121.2, 119.8, 114.1, 96.1, 84.3, 76.7, 67.0, 56.7, 53.5, 53.4, 49.3, 31.6, 31.0, 29.7, 27.7, 23.1, 15.4, 13.9, 7.4; ESI-MS, 846.24 [M + H]⁺.

### EXAMPLE 14. Boc-(10)-(7-ethyl-10-hydroxycamptothecin)-(20)-Met-NH₂•HCl (compound 16):

A solution of Boc-(10)-(7-ethyl-10-hydroxycamptothecin)-(20)-Met-Bsmoc (compound **15,** 4.1 g, 4.85 mmol) and 4-piperidinopiperidine (1.06 g, 6.31 mmol) in anhydrous CH₂Cl₂ (200 mL) was stirred at room temperature for 5 hours. This mixture was then washed with 0.1 N HCl (2 x 40ml), followed by drying the organic layer over anhydrous MgSO₄. This solution was filtered, and the solvent was removed by vacuum distillation to yield 2.8 g of product with purity of about 97%, determined by HPLC. This product was further purified by trituration with ether (3 x 20 ml), and then trituration with ethyl acetate (4 x 20ml) to yield 1.54 g with purity of 97%. ¹³C NMR (75.4 MHz, CDCl₃) d: 167.2, 166.5, 156.9, 151.12, 150.9, 149.8, 146.3, 145.9, 145.8, 144.9, 131.3, 127.2, 127.0, 125.1, 119.6, 113.8, 96.7, 84.3, 78.2, 67.0, 60.4, 52.2, 49.4, 31.4, 29.6, 29.1, 27.7, 23.2, 15.1, 13.9, 7.7.

### EXAMPLE 15. ^{40k} 4arm-PEG-Met-(20)-(7-ethyl-10-hydroxycamptothecin)-(10)-Boc (compound 17):

To an anhydrous CH₂Cl₂ (80 mL) solution, Boc-(10)-(7-ethyl-10-hydroxycamptothecin)-(20)-Met (compound **16**, 1.48 g, 2.25 mmol) and 4arm-PEG-COOH (compound 3, 9.0 g, 0.9 mmol) were added at room temperature. The solution was cooled to 0°C, followed by addition of EDC (0.26 g, 1.35 mmol) and DMAP (0.27 g, 2.25 mmol). The mixture was stirred at 0°C for 1 hour under N₂. Then it was kept at room temperature overnight. The reaction mixture was diluted with 70 ml of CH₂Cl₂, extracted with 30 ml of 0.1 N HCl/1M NaCl aqueous solution. After the organic layer was dried with MgSO₄, the solvent was evaporated under reduced pressure. The residue was dissolved in 40 mL of CH₂Cl₂, and the crude product was precipitated with ether (300 mL). The wet solid resulting from filtration was dissolved in 270 mL of DMF/IPA at 65°C. The solution was allowed to cool down to room temperature within 2 ~ 3 hours, and the product was precipitated. Then the solid was filtered and washed with ether (2 X 400 mL). The above crystallization procedure in DMF/IPA was repeated. The wet cake was dried under vacuum below 40°C overnight to give product of 7.0 g. ¹³C NMR (75.4 MHz, CDCl₃) d:169.8, 169.6, 166.5, 156.9, 151.2, 151.1, 149.9, 147.0, 146.6, 145.0, 131.7, 127,1, 126.8, 124.9, 119.7, 113.8, 95.5, 84.1, 70.1, 69.9, 66.9, 50.7, 49.2, 31.5, 31.2, 29.6, 27.6, 23.1, 15.3, 13.9, 7.5:

### EXAMPLE 16. ^{40k} 4arm-PEG-Met-(20)-(7-ethyl-10-hydroxycamptothecin) (compound 18):

To a solution of 30% TFA in anhydrous CH₂Cl₂ (100 mL), dimethyl sulfide (2.5 mL) and 4arm-PEG-Met-(20)-(7-ethyl-10-hydroxycamptothecin)-(10)-Boc (compound 17, 6.0 g) were added at room temperature. The mixture was stirred for 3 hours, or until disappearance of starting material was confirmed by HPLC. Solvents were removed as much as possible under vacuum at 35°C. The residues were dissolved in 50mL of CH₂Cl₂, and the crude product was precipitated with ether (350ml), and filtered. The wet solid was dissolved in a mixture of DMF/IPA (60/300 mL) at 65°C. The solution was allowed to cool down to room temperature within 2 ~ 3 hours, and the product was precipitated. Then the solid was filtered and washed with ether (2 x 200 mL). The wet cake was dried under vacuum below 40°C overnight to give product of 5.1 g. ¹³C NMR (75.4 MHz, CDCl₃) d: 169.7, 166.6, 157.0, 156.3, 148.4, 147.3, 145.0, 144.4, 142.9, 131.5, 128.3, 126.4, 122.5, 118.7, 105.2, 94.7, 78.1, 67.0, 50.7, 49.2, 31.6, 31.3, 29.7, 23.0, 15.3, 13.5, 7.5; Ratio of 7-ethyl-10-hydroxycamptothecin to PEG : 2.1 % (wt).

### EXAMPLE 17. Boc-(10)-(7-ethyl-10-hydroxycamptothecin)-(20)-Sar-Boc (compound 19):

Boc-Sar-OH (432 mg, 2.287 mmol) was added to a solution of Boc-(10)-(7-ethyl-10-hydroxycamptothecin) (compound 10, 750 mg, 1.52 mmol) in 75 mL of DCM and cooled to 0 °C. DMAP (432 mg, 2.287 mmol) and EDC (837 mg, 0.686 mmol) were added and the reaction mixture was stirred from 0 °C - room temperature for 1.5 hours. Reaction mixture was then washed with 0.5% NaHCO₃ (75 mL x 2), with water (75 ml x 2) and finally washed with 0.1 N HCl (75 mL x 1). The methylene chloride layer was dried over MgSO₄ and the solvent was evaporated under vacuum and dried. Yield = 0.900 mg.(89%). The structure was confirmed by NMR.

### EXAMPLE 18. 7-ethyl-10-hydroxycamptothecin-(20)-Sar•TFA (compound 20):

Boc-(10)-(7-ethyl-10-hydroxycamptothecin)-(20)-Sar-Boc (compound **19**, 900 mg, 1.357 mmol) was added to a solution of 4 mL TFA and 16 mL DCM, and stirred at room temperature for 1 hour. The reaction mixture was evaporated with toluene at 30 °C. The residue was dissolved in 10 mL CHCl₃ and precipitated with ethyl ether. The product was filtered and dried. Yield 700 mg (1.055 mmol, 78%). ¹³C NMR (67.8 MHz, CDCl₃) δ 168.26, 167.07, 158.84, 158.71, 148.82, 147.94, 147.22, 146.34, 144.04, 131.18, 130.08, 128.97, 124.46, 119.78, 106.02, 97.23, 79.84, 79.34, 66.87, 50.84, 49.86, 31.81, 23.94, 15.47, 13.84, 8.08.

### EXAMPLE 19. TBDMS-(10)-(7-ethyl-10-hydroxycamptothecin)-(20)-Sar•HCl (compound 21):

A solution of the 7-ethyl-10-hydroxycamptothecin-(20)-Sar•TFA (compound 20, 2.17 g, 3.75 mmol, 1 eq.) in anhydrous DMF (30 mL) was diluted with 200 mL of anhydrous DCM. Et₃N (2.4 mL, 17.40 mmol, 4.5 eq.) was added followed by TBDMSCl (2.04 g, 13.53 mmol, 3.5 eq.). The reaction mixture was stirred at room temperature until HPLC showed disappearance of the starting material (approximately 1 hour). The organic layer was washed with 0.5% NaHCO₃ twice, water once, and a 0.1 N HCl solution saturated with brine twice; and then dried over MgSO₄. After filtration and evaporation of the solvent under vacuum, the resulting oil was dissolved in DCM. Addition of ether gave a solid that was filtered using a fine or medium buchner funnel (2.00 g, 87% yield). HPLC of the solid showed 96% purity. ¹H NMR and ¹³C NMR confirmed the structure. ¹H NMR (300 MHz, CD₃OD): δ 0.23 (6H, s), 0.96 (9H, s), 0.98 (3 H, t, J = 7.3 Hz), 1.30 (3 H, t, J = 7.6 Hz), 2.13-2.18 (2H, m), 2.67 (3H, s), 3.11 (2 H, q, J = 7.6 Hz), 4.10 (1H, d, J = 17.6 Hz), 4.22 (1H, d, J = 17.6 Hz), 5.23 (2 H, s), 5.40 (1 H, d, J = 16.7 Hz), 5.55 (1H, d, J = 16.7 Hz), 7.32 (1H, s), 7.38-7.43 (2H, m), 8.00 (1H, d, J = 9.1 Hz). ¹³C NMR (75.4 MHz, CD₃OD): δ -4.14, 8.01, 14.10, 19.30, 23.98, 26.16, 31.78, 33.52, 49.46, 50.95, 67.66, 79.80, 97.41, 111.96, 119.99, 127.75, 129.28, 129.67, 131.57, 145.24, 146.86, 147.16, 148.02, 150.34, 156.69, 158.72, 167.02, 168.27.

### EXAMPLE 20. ^{40K} 4arm-PEG-Sar-(20)-(7-ethyl-10-hydroxycamptothecin)-(10)-TBDMS (compound 22):

To a solution of ^{40K} 4arm-PEG-COOH (compound 3, 10 g, 0.25 mmol, 1 eq.) in 150 mL of anhydrous DCM was added a solution of TBDMS-(10)-(7-ethyl-10-hydroxycamptothecin)-Sar•HCl (compound **21**, 1.53 g, 2.5 mmol, 2.5 eq.) in 20 mL of anhydrous DMF and the mixture was cooled to 0 °C. To this solution were added EDC (767 mg, 4 mmol, 4 eq.) and DMAP (367 mg, 3 mmol, 3 eq.) and the reaction mixture was allowed to warm to room temperature slowly and stirred at room temperature overnight. Then, the reaction mixture was evaporated under vacuum and the residue was dissolved in a minimum amount of DCM. After addition of ether, solid was formed and filtered under vacuum. The residue was dissolved in 30 mL of anhydrous CH₃CN and precipitated by addition of 600 mL IPA. The solid was filtered and washed with IPA and ether to give the product (9.5 g). The structure was confirmed by NMR.

### EXAMPLE 21. ^{40K} 4arm-PEG-Sar-(20)-(7-ethy-10-hydroxycamptothecin) (compound 23):

**Method A.** ^{40K} 4arm-PEG-Sar-(20)-(7-ethyl-10-hydroxycamptothecin)-(10) TBDMS (compound 22) was dissolved in a 50% mixture of TFA in H₂O (200 mL). The reaction mixture was stirred at room temperature for 10 hours and then, diluted with 100 mL of H₂O and extracted with DCM (2 x 300 mL). The combined organic phases were washed with H₂O (2 x 100 mL), dried over MgSO₄, filtered and evaporated under vacuum. The residue was dissolved in 100 mL of anhydrous DMF gently heated with a heat gun and precipitated by slow addition of 400 mL DMF. The solid was filtered and washed with 20% DMF in IPA and ether. The solid was dissolved in DCM and precipitated with ether (6.8 g). The structure was conformed by NMR.

**Method B.** ^{40K} 4arm-PEG-Sar-(20)-(7-ethyl-10-hydroxycamptothecin)-(10)-TBDMS (1 g) was dissolved in 10 mL of a 1N HCl solution. The reaction mixture was stirred at room temperature for 1 hour (checked by HPLC) and then extracted with DCM (2 x 40 mL). The organic layers were dried over MgSO₄, filtered and evaporated under vacuum. The resulting bright yellow residue was dissolved in 10 mL of DMF (slightly heated with a heat gun) and then 40 mL of IPA were added. The resulting solid was filtered and dried overnight at 40 °C in a vacuum oven. The structure was confirmed by NMR.

### BIOLOGICAL DATA

### EXAMPLE 22. TOXICITY DATA

A maximum tolerated dose (MTD) of four-arm PEG conjugated 7-ethyl-10-hydroxycamptothecin was studied using nude mice. Mice were monitored for 14 days for mortality and signs of illness and sacrificed when body weight loss was >20% of the pretreatment body weight.

Table 1 shows the maximum tolerated dose of each compound for both single dose and multiple dose administration. Each dose for multiple dose administration was given mice every other day for 10 days and the mice were observed for another 4 days, thus for total 14 days.

**TABLE 1. MTD Data in Nude Mice**

| **Compound** | **Dose Level (mg/kg)** | **Survival/Total** | **Comments** |
|---|---|---|---|
| Compound 9 Single dose | 25 | 5/5 | |
| | 30 | 5/5 | |
| | 35 | 4/5 | Mouse euthanized due to >20% body weight loss |
| Compound 9 Multiple dose* | 10 | 5/5 | |
| | 15 | 3/5 | Mice euthanized due to >20% body weight loss |
| | 20 | 0/5 | Mice euthanized due to >20% body weight loss |

The MTD found for 4arm-PEG-Gly-(7-ethyl-10-hydroxycamptothecin) (compound 9) was 30 mg/kg when given as single dose, and 10 mg/kg when given as multiple dose (q2d x 5).

### EXAMPLE 23. In Vitro DATA

In Table 2, the cytotoxicity (µM of each compound that results in an IC₅₀) provides an indication of the *in vitro* anti-tumor potency of each compound. This study was used to determine the effect of PEGylation of four-arm PEGylated 7-ethyl-10-hydroxycamptothecin conjugates. The *in vitro* cytotoxicity of PEG-Gly-(7-ethyl-10-hydroxycamptothecin), 7-ethyl-10-hydroxycamptothecin, and CPT-11 was determined using a MTS assay. Cells were incubated with drugs for 72 hours at 37 °C. Following incubation, MTS dye was added and formation of a colored product (formazan) was measured at 490nm.

The IC₅₀ values of four-arm PEG-Gly-(7-ethyl-10-hydroxycamptothecin) and CPT-11 indicate that four-arm PEG-Gly-(7-ethyl-10-hydroxycamptothecin) have much higher *in vitro* inhibition in all tested tumor cells than CPT-11. In addition, four-arm PEG-Gly-(7-ethyl-10-hydroxycamptothecin) has significantly *higher in vitro* inhibition of the tested tumor cells except for HT29 tumor cells than native 7-ethyl-10-hydroxycamptothecin.

PEG-Gly-(7-ethyl-10-hydroxycamptothecin) conjugates were about 10 to 600 fold more potent than Camptosar®. PEG-Gly-(7-ethyl-10-hydroxycamptothecin) conjugates were 8 to 16 fold more sensitive than Pegamotecan (a PEGylated prodrug of camptothecin) in COLO 205, HT-29 and OVCAR-3 cell lines.

**TABLE 2. IC₅₀ (µM) data**

| **Cancer Type** | **Cell Line** | **7-ethyl-10-hydroxycamptothecin** | **Compound 9** | **CPT-11** |
|---|---|---|---|---|
| **Colorectal** | Colo 205 | 0.2 ± 0.2 | 1.0 ± 0.6 | 11 ± 1.4 |
| | HT29 | 0.1 ± 0.04 | 0.5 ± 0.3 | 27 ± 8.6 |
| **Lung** | A549 | 1.0 ± 0.1 | 2.7 ± 0.4 | 67 ± 17 |
| **Pancreatic** | PANC-1 | 0.56 ± 0.38 | 0.67 ± 0.14 | 34 ± 16 |
| | MIA PaCa-2 | 0.18 | 0.09 | 67 ± 46 |
| | ASPC-1 | 0.64 ± 0.13 | 1.6 ± 1.1 | 33 ± 1.0 |
| | BxPC-3 | 0.54 ± 0.58 | 0.24 ± 0.04 | 44 ± 44 |
| **Ovarian** | OVCAR-3 | 0.1 ± 0.02 | 0.3 ± 0.2 | 20 ± 7.1 |
| | OV90 | 0.1 ± 0.02 | 0.6 ± 0.7 | 18 ± 4.8 |
| | OVCAR-8 | 0.03 ± 0.01 | 0.5 ± 0.1 | 9.0 ± 1.3 |
| | A2780 | 0.02 ± 0.01 | 0.2 ± 0.2 | 8.0 ± 3.5 |
| | SK-0V-3 | 0.2 ± 0.1 | 0.9 ± 0.01 | 52 ± 8.5 |

| | | | | |
|---|---|---|---|---|
| IC₅₀ of 4armPEG-Gly-(7-ethyl-10-hydroxycamptothecin) (compound 9) is expressed in terms of 7-ethyl-10-hydroxycamptothecin equivalents. Values shown are average ± SD (n=3); where no SD is shown, the value shown is an average of two replicates. | | | | |

PEG-(7-ethyl-10-hydroxycamptothecin) conjugates containing various amino acids are potent *in vitro* cytotoxicity against a panel of cancer cell lines as summarized in TABLE 3. All the PEG-7-ethyl-10-hydroxycamptothecin conjugates showed potent *in vitro* anti-tumor activities which exceed the activity of small molecule prodrug CPT-11 and Pegamotecan.

**TABLE 3. IC₅₀ (µM) data of Various PEG-(7-ethyl-10-hydroxycamptothecin) Conjugates**

| | **Colorectal** | | **Ovarian** | **Lung** |
|---|---|---|---|---|
| **Compound** | **Colo 205** | **HT29** | **OVCAR-3** | **A549** |
| Compound 12 (Ala) | 0.03 | 0.16 | 0.14 | 4.4 |
| Compound 23 (Sar) | 0.04 | 0.27 | 0.17 | 11 |
| Compound 18 (Met) | 0.03 | 0.14 | 0.13 | 1.6 |
| 7-ethyl-10-hydroxycamptothecin | 0.08 | 0.08 | ND | ND |
| CPT-11 | 20 | 56 | 41 | 13 |

### EXAMPLE 24. In Vivo DATA-Single Dose Efficacy in Breast Tumor Xenograft Mice

The efficacy of four-arm PEG-Gly-(7-ethyl-10-hydroxycamptothecin) conjugates (compound 9) against a subcutaneous human mammary carcinoma (MX-1) grown in nude mice was determined as follows. Following at least one week of acclimation, tumors were established by implanting small tumor fragments from donor mice into a single subcutaneous site, on the left auxiliary flank region of nude mice. The tumor implantation site was observed twice weekly and measured once palpable. The tumor volume for each mouse was determined by measuring two dimensions with calipers and calculated using the formula: tumor volume = (length x width²). When tumors reached the average volume of approximately 100mm³, the mice were divided into their experimental groups, which consists of untreated controls, four-arm PEG-Gly-(7-ethyl-10-hydroxycamptothecin) made as per Example 7 herein, PEG-Ala-CPT prepared as described in Conover et al. (Anti-Cancer Drug Design, 1999, 14:499-506), the contents of which are incorporated by reference herein and CPT-11. Drugs were administered intravenously via the tail vein as a single dose of 20 mg/kg body weight of a mouse. The dose of the conjugate refers to the amount equivalent to 7-ethyl-10-hydroxycamptothecin. Mouse weight and tumor sizes were measured at the beginning of study and on the 31^{st} day after the single dose administration.

Table 4 shows tumor shrinkages measured in mice xenografted with MX-1 breast tumor cells on the 31^{st} day after a single dose i.v. injection of 20 mg/kg body weight of a mouse. The overall growth of tumors was calculated as the mean tumor volume (TV). A percent treatment over control (T/C) was also calculated. The percentage of tumor regression indicates the *in vivo* activity of each compound. Four-arm PEG-GLY-7-ethyl-10-hydroxycamptothecin conjugates showed significant anti-tumor activity with 100% survival of the mice and 100% cure. Both CPT-11 and saline control showed no protection with 0% survival. All the data are summarized in Table 4.

**TABLE 4. Single Dose Efficacy Comparison in MX-1 Breast Tumor Xenograft Mouse Model**

| **Compound** | **Mean TV ± SD** | **Median TV** | **F/I** | **Chg in TV±SD** | **TGI** | **Regressions** | **Cures** | **Survival** | **T/C** |
|---|---|---|---|---|---|---|---|---|---|
| | **(mm³)** | **(mm³)** | **(%)** | **(%)** | **(%)** | **(%)** | **(%)** | **(%)** | **(%)** |
| Control | 1136 ± 686 | 785 | 1259 | 1399 ± 658 | 0 | 0 | 0 | 0 | --- |
| Compound 9 | 7 ± 9 | 3 | 7 | -94 ± 10 | 99 | 100 | 100 | 100 | 0.3 |
| PEG-Ala-CPT | 43 ± 41 | 40 | 42 | -40 ± 93 | 96 | 50 | 33 | 100 | 5 |
| CPT-11 | 845 ± 50 | 845 | 843 | 1424 ± 432 | 26 | 0 | 0 | 0 | 108 |

The results show that the four-arm PEG-Gly-(7-ethyl-10-hydroxycamptothecin) is significantly more effective than either PEG-Ala-CPT or CPT-11 in the treatment of breast cancer.

In breast MX-1 tumor model, treatment with 4armPEG-Gly-(7-ethyl-10-hydroxycamptothecin) led to almost 100% tumor growth inhibition and complete cures of all the animals. At equivalent dose levels, treatment with CPT-11 caused a 26 % tumor growth inhibition.

### EXAMPLE 25. In Vivo DATA-Multiple Dose Efficacy in Breast Tumor Xenograft Mice

The efficacy of a series of four-arm PEG-Amino acid (derivative)-(7-ethyl-10-hydroxycamptothecin) conjugates against a human mammary carcinoma (MX-1) grown in nude mice was determined. The mice were assigned to groups each receiving 4arm-PEG-Ala-(7-ethyl-10-hydroxycamptothecin), 4arm-PEG-Gly-(7-ethyl-10-hydroxycamptothecin), 4arm-PEG-Met-(7-ethyl-10-hydroxycamptothecin), 4arm-PEG-Sar-(7-ethyl-10-hydroxycamptothecin), each made as described herein, CPT-11 or saline control. The mice were dosed 5 mg/kg/day for six consecutive days (every 2 days [qx2d] x 6). Mouse weight and tumor sizes were monitored up to the 31^{st} day after the first dose administration and subsequent administrations.

Table 5 shows tumor shrinkages measured in mice xenografted with MX-1 breast tumor cells on the 31^{st} day after the i.v. injection of 5 mg/kg/dose for six days (q2d x 6). All four-arm PEG-Amino acid (derivative)-(7-ethyl-10-hydroxycamptothecin) conjugates showed significant anti-tumor activity with 100% survival of the mice and 100% regression. Both CPT-11 and saline control showed no protection with 0% survival. All the data are summarized in Table 5.

**TABLE 5. Multiple Dose Efficacy Comparison in MX-1 Breast Tumor Xenograft Mouse Model**

| Compound | **Mean TV ± SD** | **Median TV** | **F/I** | **Chg in TV±SD** | **TGI** | **Regressions** | **Cures** | **Survival** | **T/C** |
|---|---|---|---|---|---|---|---|---|---|
| | **(mm³)** | **(mm³)** | **(%)** | **(%)** | **(%)** | **(%)** | **(%)** | **(%)** | **(%)** |
| Control | 1136 ± 686 | 785 | 1259 | 1399 ± 658 | 0 | 0 | 0 | 0 | --- |
| Compound 12 (Ala) | 10 ± 11 | 9.6 | 10 | -91 ± 11 | 99 | 100 | 67 | 100 | 1.2 |
| Compound 9 (Gly) | 12 ± 18 | 6.6 | 14 | -91 ± 10 | 99 | 100 | 100 | 100 | 0.8 |
| Compound 18 (Met) | 8 ± 9 | 5.6 | 7.8 | -94 ± 7 | 99 | 100 | 100 | 100 | 0.7 |
| Compound 23 (Sar) | 12 ± 10 | 14.6 | 13 | -87 ± 12 | 99 | 100 | 100 | 100 | 1.9 |
| CPT-11 | 632 ± 698 | 340 | 594 | 423 ± 243 | 44 | 0 | 0 | 0 | 43 |

The data shows that the four-arm PEG-Amino acid derivatives of 7-ethyl-10-hydroxycamptothecin are significantly more effective than CPT-11 in the treatment of breast cancer.

### EXAMPLE 26. In Vivo Efficacies in Small and Large Breast Tumor Xenografted Mice

The antitumor efficacy of 4armPEG-Gly-(7-ethyl-10-hydroxycamptothecin) was evaluated in nude mice bearing small (about 100 mm³) or large (about 450 mm³) established human mammary, MX-1 tumors. The antitumor therapeutic activities were determined in mice both with a single dose or multiple dose regimens. In mice xenografted with small breast tumors, treatment with a single of 4armPEG-Gly-(7-ethyl-10-hydroxycamptothecin) (20mg/kg) led to 100% TGI. Treatment with multiple doses of 4armPEG-Gly-(7-ethyl-10-hydroxycamptothecin) (20mg/kg q2d x 5) also led to 100% TGI. Single dose (20mg/kg) administration of CPT-11 did not inhibit tumor growth and multiple doses of CPT-11 (20mg/kg q2d x 5) resulted in 44% TGI as of day 31.

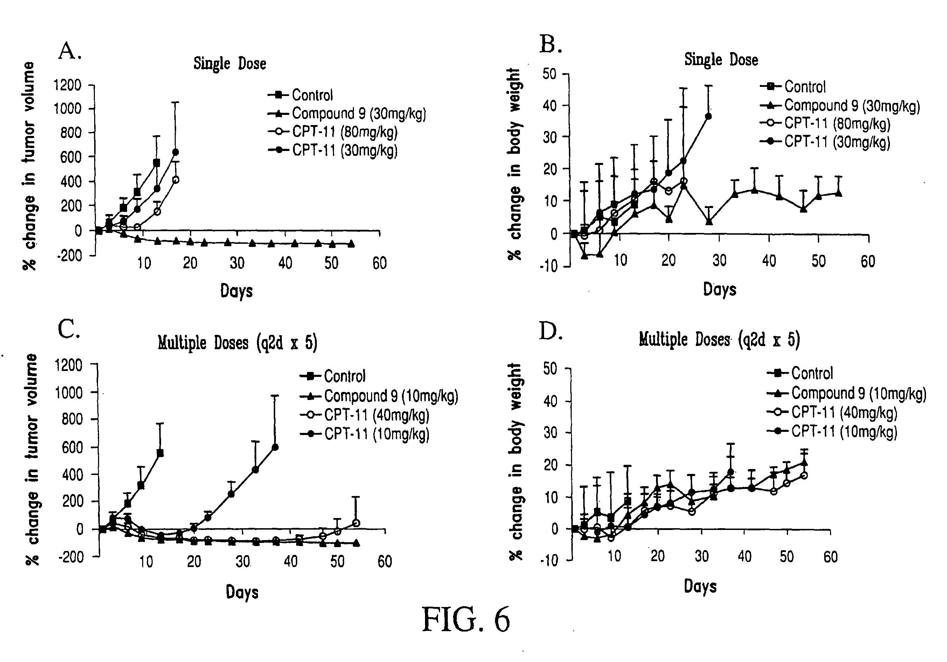

In mice bearing large breast tumors, treatment with a single MTD of 4armPEG-Gly-(7-ethyl-10-hydroxycamptothecin) (30 mg/kg) led to 96% TGI. Four of six mice were cured as of day 54. Treatment with a single MTD of CPT-11 (80mg/kg) resulted in 70% TGI on day 13. All animals were sacrificed by day 20 due to excessive tumor burden. Treatment with multiple doses of 4armPEG-Gly-(7-ethyl-10-hydroxycamptothecin) at its MTD (10 mg/kg q2d x 5) also led to 96% TGI, and five of six mice were cured as of day 54. CPT-11, when given as multiple doses at its MTD (40mg/kg q2d x 5), led to ∼95% TGI as of day 13. However, tumors regrew in four of the six mice after ~5 weeks. CPT-11, when given at equivalent dose levels as 4armPEG-Gly-(7-ethyl-10-hydroxycamptothecin) (10 mg/kg q2d x 5), had effective TGI until treatment was discontinued, after which tumors regrew. The results are set forth in Figures. 6A and 6C. The antitumor effects are not attributed to a general toxic effect, since treatment with either 4armPEG-Gly-(7-ethyl-10-hydroxy-camptothecin) or CPT-11, when given at the MTD, did not significantly decrease the body weight of the animals for the duration of the treatment as shown in Figures 6B and 6D.

In MX-1 breast tumor model with a single dose or multiple dose injections, 4armPEG-Gly-(7-ethyl-10-hydroxycamptothecin) effectively suppressed tumor growth in both small (100 mm³) and large (450 mm³) bulky established tumors. Tumors in size of from about 75 mm³ to about 450 mm³ were successfully treated in MX-1 breast tumor xenografted mice.

### EXAMPLE 27. In Vivo DATA-Single Dose Efficacy in Colorectal Tumor Xenograft Mice

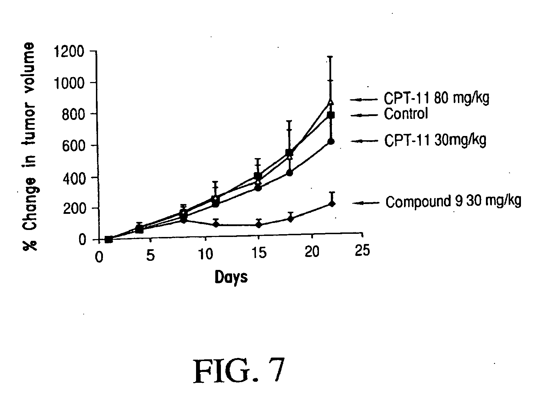

In this example, tumor shrinkage was measured in mice xenografted with HT-29 colorectal tumor cells on the day 4, 8, 11, 15, 18, and 22 after a single dose intravenous injection of 30 mg/kg of four-arm PEG-Gly-(7-ethyl-10-hydroxycamptothecin) or CPT-11. CPT 11 was also dosed 80 mg/kg. The results are set forth in Fig. 7. Four-arm PEG-Gly-(7-ethyl-10-hydroxycamptothecin) conjugates showed significant anti-tumor activity. Saline control showed no protection with 0% survival. The results show that the four-arm PEG-Gly-(7-ethyl-10-hydroxycamptothecin) is significantly effective than CPT-11 in the treatment of colorectal cancer.

### EXAMPLE 28. In Vivo DATA-Multiple Dose Efficacy in Colorectal Tumor Xenograft Mice

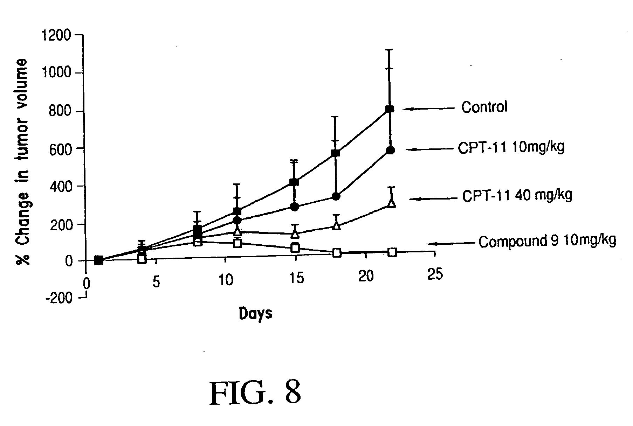

The tumor shrinkage was measured in mice xenografted with HT-29 colorectal tumor cells on the day 4, 8, 11, 15, 18, and 22 after an intravenous injection of 10 mg/kg/dose of four-arm PEG-Gly-(7-ethyl-10-hydroxycamptothecin) or CPT-11 for five days. CPT-11 was also given 40 mg/kg/dose for five days. The results are set forth in Fig. 8. Similar results were obtained with multiple dose administration. Furthermore, multiple dose administration of four-arm PEG-Gly-(7-ethyl-10-hydroxycamptothecin) shows significant therapeutic efficacy compared to that of CPT-11. The results show that multiple doses of four-arm PEG-Gly-(7-ethyl-10-hydroxycamptothecin) is significantly more effective than CPT-11 in the treatment of colorectal cancer. As observed in the MX-1 tumor xenografted mice, treatment with 4armPEG-Gly-(7-ethyl-10-hydroxycamptothecin) or CPT-11 did not significantly decrease the body of weight of the mice during the treatment.

### EXAMPLE 29. In Vivo DATA-Single Dose Efficacy in Pancreatic Tumor Xenograft Mice

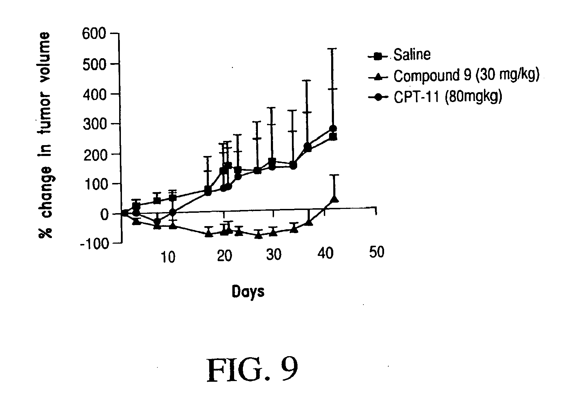

In this example, tumor shrinkage was measured in mice xenografted with MiaPaCa-2 pancreatic tumor cells on the day 4, 8, 11, 15, 18, and 22 after a single dose intravenous injection of 30 mg/kg of four-arm PEG-Gly-(7-ethyl-10-hydroxycamptothecin) or CPT-11. CPT 11 was also dosed 80 mg/kg. The results are set forth in Fig. 9. Four-arm PEG-Gly-(7-ethyl-10-hydroxycamptothecin) conjugates showed significant antitumor activity. Saline control and CPT-11 showed no protection. The results show that the four-arm PEG-Gly-(7-ethyl-10-hydroxycamptothecin) is significantly effective than CPT-11 in the treatment of pancreatic cancer. Treatment with a single does of 4armPEG-Gly-(7-ethyl-10-hydroxycamptothecin) resulted in 71 % TGI, whereas CPT-11 therapy as a single dose injection caused 0 % TGI.

### EXAMPLE 30. In Vivo DATA-Multiple Dose Efficacy in Pancreatic Tumor Xenograft Mice

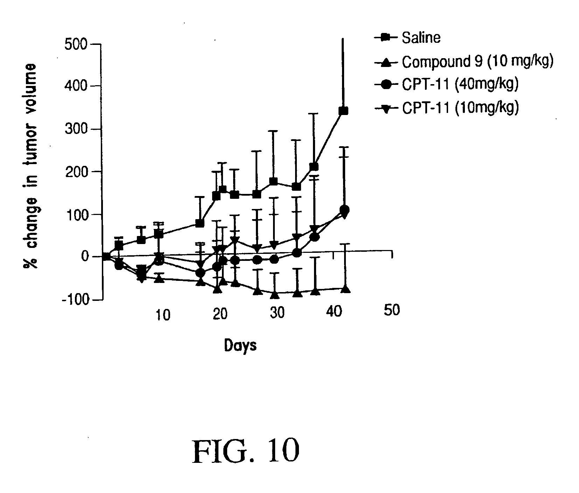

The tumor shrinkage was measured in mice xenografted with MiaPaCA-2 pancreatic tumor cells on the day 4, 8, 11, 15, 18, and 22 after an intravenous injection of 10 mg/kg/dose of four-arm PEG-Gly-(7-ethyl-10-hydroxycamptothecin) or CPT-11 for five days. CPT-11 was also given 40 mg/kg/dose for five days. The results are set forth in Fig. 10. Multiple dose administration of four-arm PEG-Gly-(7-ethyl-10-hydroxycamptothecin) shows significant therapeutic efficacy compared to that of CPT-11. The results show that multiple doses of four-arm PEG-Gly-(7-ethyl-10-hydroxycamptothecin) is more effective than CPT-11 in the treatment of pancreatic cancer. Treatment with multiple doses of 4armPEG-Gly-(7-ethyl-10-hydroxycamptothecin) resulted in 95% TGI, whereas CPT-11 caused 34% TGI.

### EXAMPLE 31. In Vitro Metabolism

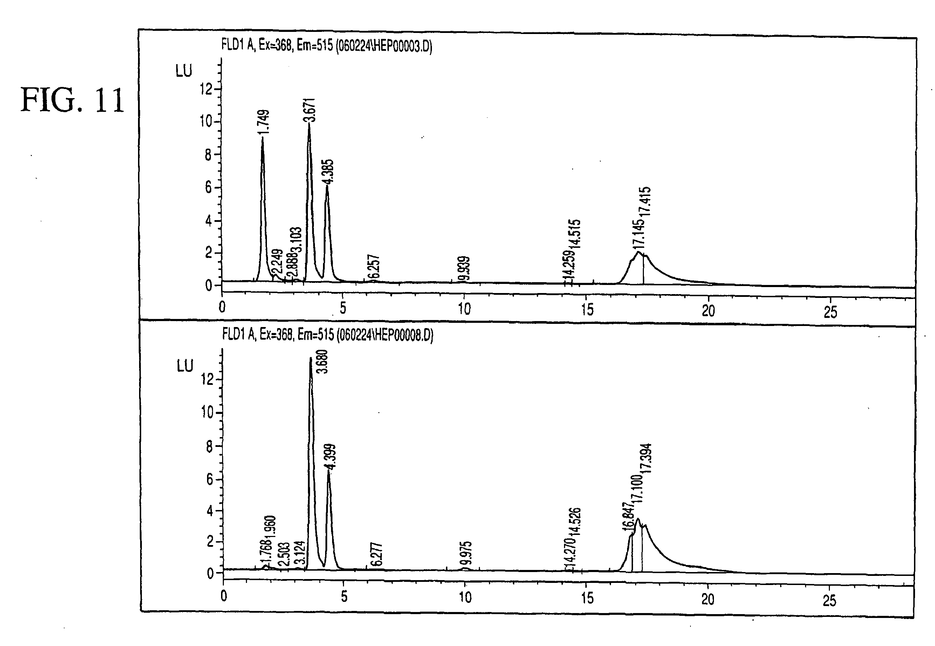

In vitro metabolism of PEG-Gly-(7-ethyl-10-hydroxycamptothecin) conjugates was observed in rat hepatocytes. PEG-Gly-(7-ethyl-10-hydroxycamptothecin) was incubated with rat hepatocytes for 2 hours, pH 7.5, 37 °C. As shown in Figure 11, 7-ethyl-10-hydroxycamptothecin and 7-ethyl-10-hydroxycamptothecin-Glucuronide (7-ethyl-10-hydroxycamptothecin-G) were the major metabolites identified, which agrees with known metabolic pathway of 7-ethyl-10-hydroxycamptothecin *in vivo.*

### EXAMPLE 32. Properties of PEG Conjugates

Table 6 shows solubility of four different PEG-(7-ethyl-10-hydroxycamptothecin) conjugates in aqueous saline solution. All four PEG-(7-ethyl-10-hydroxycamptothecin) conjugates showed good solubility of up to 4 mg/mL equivalent of 7-ethyl-10-hydroxycamptothecin. In human plasma, 7-ethyl-10-hydroxycamptothecin was steadily released from the PEG conjugates with a doubling time of 22 to 52 minutes and the release appeared to be pH and concentration dependent as described in the following EXAMPLE 33.

**TABLE 6. Properties of PEG-7-ethyl-10-hydroxycamptothecin Conjugates**

| **Compound** | **Solubility in Saline (mg/mL)^{a}** | **t _{1/2}(min) in Human Plasma^{b}** | **Doubling Time in Plasma (min)^{c}** | | |
|---|---|---|---|---|---|
| | | | **Human** | **Mouse** | **Rat** |
| Compound 9 (Gly) | 180 | 12.3 | 31.4 | 49.5 | 570 |
| Compound 12 (Ala) | 121 | 12.5 | 51.9 | 45.8 | 753 |
| Compound 23 (Sar) | ND | 19.0 | 28.8 | 43.4 | 481 |
| Compound 18 (Met) | 142 | 26.8 | 22.2 | 41.9 | 1920 |

| | | | | | |
|---|---|---|---|---|---|
| ^{a} 7-ethyl-10-hydroxycamptothecin is not soluble in saline. ^{b} PEG conjugate half life. ^{c} 7-ethyl-10-hydroxycamptothecin formation rate from conjugates. | | | | | |

PEG-7-ethyl-10-hydroxycamptothecin conjugates show good stability in saline and other aqueous medium for up to 24 hours at room temperature.

### EXAMPLE 33. Effects Of Concentration and pH on Stability

Based on our previous work, acylation at the 20-OH position protects the lactone ring in the active closed form. The aqueous stability and hydrolysis properties in rat and human plasma were monitored using UV based HPLC methods. 4armPEG-Gly-(7-ethyl-10-hydroxycamptothecin) conjugates were incubated with each sample for 5 minutes at room temperature.

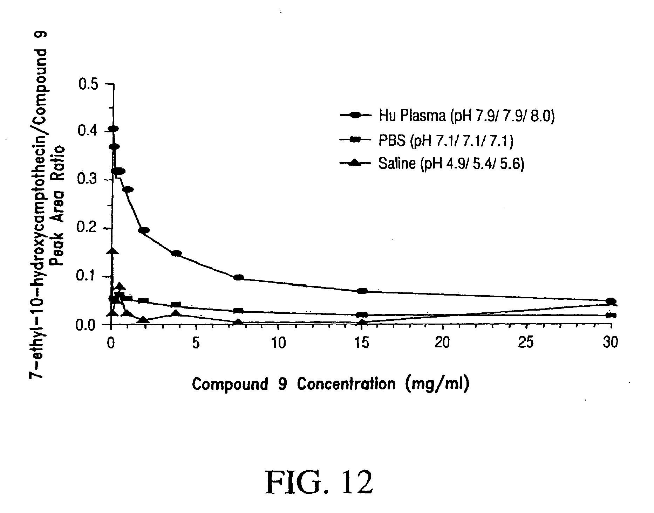

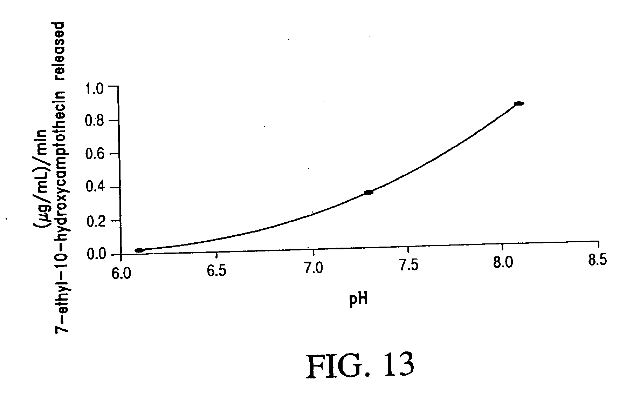

Stability of PEG-7-ethyl-10-hydroxycamptothecin conjugates in buffer was pH dependent. Figure 12 shows 4armPEG-Gly-(7-ethyl-10-hydroxycamptothecin) stability in various samples. Figure 13 shows that rate of 7-ethyl-10-hydroxycamptothecin release from PEG-Gly-(7-ethyl-10-hydroxycamptothecin) increases with increased pH.

### EXAMPLE 34. Pharmacokinetics

Tumor free Balb/C mice were injected with a single injection of 20 mg/kg 4armPEG-Gly-(7-ethyl-10-hydroxycamptothecin) conjugates. At various time points mice were sacrificed and plasma was analyzed for intact conjugates and released 7-ethyl-10-hydroxycamptothecin by HPLC. Pharmacokinetic analysis was done using non-compartmental analysis (WinNonlin). Details are set forth in Table 7.

**Table 7. Pharmacokinetic Data**

| **Parameter** | **Compound 9** | **7-ethyl-10-hydroxy-camptothecin Released from Compound 9** |
|---|---|---|
| **AUC (h*µg/mL)** | 124,000 | 98.3 |
| **Terminal t_{1/2} (Hr)** | 19.3 | 14.2 |
| **Cₘₐₓ (µg/mL)** | 20,500 | 13.2 |
| **CL(mL/hr/kg)** | 5.3 | 202 |
| **Vss (mL/kg)** | 131 | 3094 |

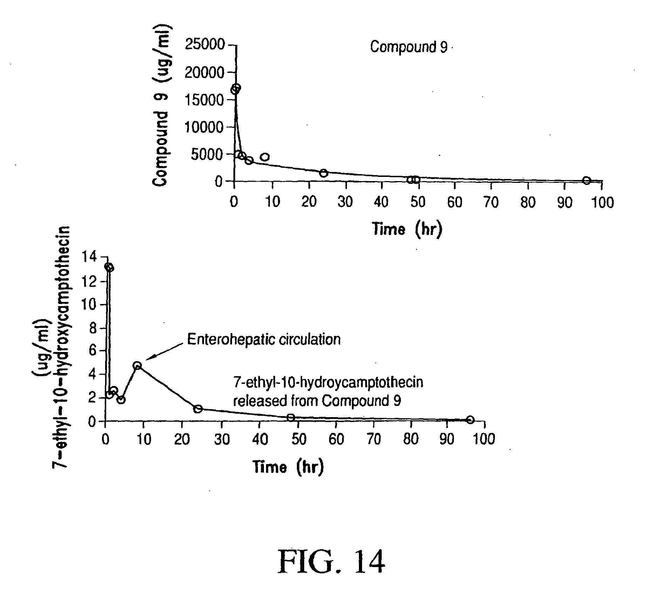

As shown in Figure 14, pegylation of 7-ethyl-10-hydroxycamptothecin allows long circulation half life and high exposure to native drug 7-ethyl-10-hydroxycamptothecin. Enterohepatic circulation of 4armPEG-Gly-(7-ethyl-10-hydroxycamptothecin) conjugates was observed. The pharmacokinetic profile of PEG-Gly-(7-ethyl-10-hydroxycamptothecin) in mice was biphasic showing a rapid plasma distribution phase during the initial 2 hours followed by a 18-22 hours terminal elimination half-life for the conjugate and a concomitant 18-26 hours terminal elimination half-life for 7-ethyl-10-hydroxycamptothecin.

Additionally, pharmacokinetic profiles of 4arm PEG-Gly-(7-ethyl-10-hydroxycamptothecin) were investigated in rats. In rats, does levels of 3, 10 and 30 mg/kg (7-ethyl-10-hydroxycamptothecin equivalent) were used. The pharmacokinetic profiles in rats were consistent with those of mice.

In rats, 4arm PEG-Gly-(7-ethyl-10-hydroxycamptothecin) showed a biphasic clearance from the circulation with an elimination half life of 12-18 hours in rats. 7-ethyl-10-hydroxycamptothecin released from 4armPEG-Gly-7-ethyl-10-hydroxycamptothecin conjugates had an apparent elimination half life of 21-22 hours. The maximum plasma concentration (Cₘₐₓ) and area under the curve (AUC) increased in a dose dependent manner in rats. The apparent half life of released 7-ethyl-10-hydroxycamptothecin from 4armPEG-Gly conjugates in mice or rats is significantly longer than the reported apparent half life of released 7-ethyl-10-hydroxycamptothecin from CPT-11 and the exposure of released 7-ethyl-10-hydroxycamptothecin from 4arm PEG-Gly-(7-ethyl-10-hydroxycamptothecin) is significantly higher than the reported exposure of released 7-ethyl-10-hydroxycamptothecin from CPT-11. The clearance of the parent compound was 0.35 mL/hr/kg in rats. The estimated volume of distribution at steady state (Vss) of the parent compound was 5.49 mL/kg. The clearance of the released 7-ethyl-10-hydroxycamptothecin was 131 mL/hr/kg in rats. The estimated Vss of released 7-ethyl-10-hydroxycamptothecin was 2384 mL/kg in rats. Enterohepatic circulation of released 7-ethyl-10-hydroxycamptothecin was observed both in mice and rats.

## Claims

1. A compound of the Formula (I) 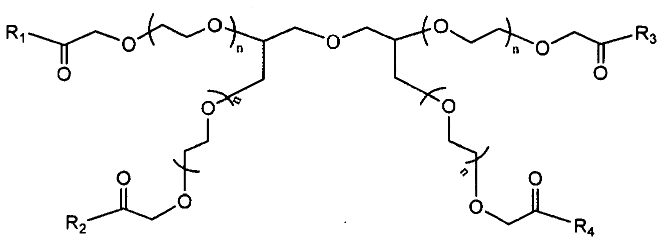 wherein
R₁, R₂, R₃ and R₄ are independently OH or (L)ₘ-D;
L is a residue of an amino acid or amino acid derivative, wherein the amino acid derivative is selected from the group consisting of
2-aminoadipic acid, 3-aminoadipic acid, beta-alanine, beta-aminopropionic acid, 2-aminobutyric acid, 4-aminobutyric acid, piperidinic acid, 6-aminocaproic acid, 2-aminoheptanoic acid, 2-aminoisobutyric acid, 3-aminoisobutyric acid, 2-aminopimelic acid, 2,4-aminobutyric acid, desmosine, 2,2-diaminopimelic acid, 2,3-diaminopropionic acid, n-ethylglycine, N-ethylasparagine, 3-hydroxyproline, 4-hydroxyproline, isodesmosine, allo-isoleucine, N-methylglycine, sarcosine, N-methyl-isoleucine, 6-N-methyl-lysine, N-methylvaline, norvaline, norleucine, and ornithine; or
L is a bifunctional linker selected from the group consisting of
-[C(O)]ᵥNR₂₁(CR₂₂R₂₃O)ₜ-
-[C(O)]ᵥNR₂₁(CR₂₂R₂₃O)ₜ(CR₂₄R₂₅)_{y}O-,
-[C(O)]ᵥNR₂₁(CR₂₂R₂₃O)ₜ(CR₂₄R₂₅)_{y}-,
-[C(O)]ᵥNR₂₁(CR₂₂R₂₃)ₜO-,
-[C(O)]ᵥNR₂₁(CR₂₂R₂₃)ₜ(CR₂₄CR₂₅O)_{y}NR₂₆-,
-[C(O)]ᵥO(CR₂₂R₂₃)ₜNR₂₆-,
-[C(O)]ᵥO(CR₂₂R₂₃)ₜO-,
-[C(O)]ᵥNR₂₁(CR₂₂R₂₃)ₜNR₂₆-,
-[C(O)]ᵥNR₂₁(CR₂₂R₂₃)ₜ(CR₂₄CR₂₅O)_{y}-,
-[C(O)]ᵥNR₂₁(CR₂₂R₂₃O)ₜ(CR₂₄CR₂₅)_{y}NR₂₆-, 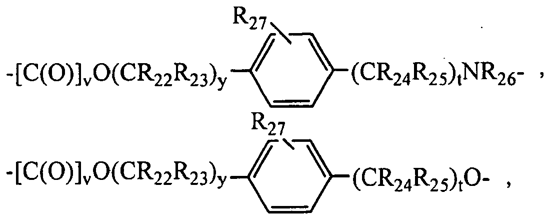 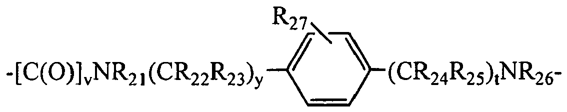
and 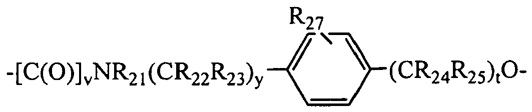 wherein:
R₂₁-R₂₆ are independently selected from the group consisting of hydrogen, C₁₋₆ alkyls, C₂₋₆ alkenyls, C₂₋₆ alkynyls, C₃₋₁₉ branched alkyls, C₃₋₈ cycloalkyls, C₁₋₆ substituted alkyls, C₂₋₆ substituted alkenyls, C₂₋₆ substituted alkynyls, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy and C₁₋₆ heteroalkoxy;
R₂₇ is selected from the group consisting of hydrogen, C₁₋₆ alkyls, C₂₋₆ alkenyls, C₂₋₆ alkynyls, C₃₋₁₉ branched alkyls, C₃₋₈ cycloalkyls, C₁₋₆ substituted alkyls, C₂₋₆ substituted alkenyls, C₂₋₆ substituted alkynyls, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy, C₁₋₆ heteroalkoxy, NO₂, haloalkyl and halogen;
t and y are individually selected positive integers from about 1 to about 4; and
v is 0 or 1;
m is a positive integer from 1 to 10;
n is a positive integer;
provided that R₁, R₂, R₃ and R₄ are not all OH; and
D is a residue of a camptothecin or of the compound of the Formula (II) 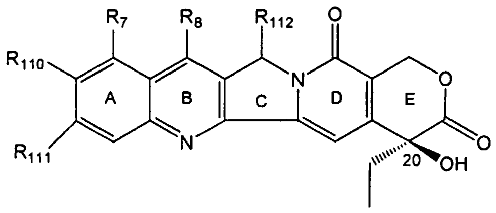 wherein
R₇ is selected from the group consisting of NO₂, NH₂, N₃, hydrogen, halogen, F, Cl, Br, I, COOH, OH, O-C₁₋₈ alkyl, SH, S-C₁₋₃ alkyl, CN, CH₂NH₂, NH-C₁₋₃ alkyl, CH₂-NH-C₁₋₃ alkyl, N(C₁₋₃ alkyl)₂, CH₂N(C₁₋₃alkyl), O-, NH- and S-CH₂CH₂N(CH₂CH₂OH)₂, O-, NH- and S-CH₂CH₂CH₂N(CH₂CH₂OH)₂, O-, NH- and S-CH₂CH₂N(CH₂CH₂CH₂OH)₂, O-, NH- and S-CH₂CH₂CH₂N(CH₂CH₂CH₂OH₂)₂, O-, NH-and S-CH₂CH₂N(C₁₋₃ alkyl)₂, O-, NH- and S-CH₂CH₂CH₂N(C₁₋₃ alkyl)₂, CHO and C₁₋₃ alkyl; and
R₈ is selected from the group consisting of H, C₁₋₈ alkyl and CH₂NR₉R₁₀
wherein
R₉ is selected from the group consisting of hydrogen, C₁₋₆ alkyl, C₃₋₇ cycloalkyl, C₃₋₇ cycloalkyl-C₁₋₆ alkyl, C₂₋₆ alkenyl, hydroxy-C₁₋₆ alkyl, and C₁₋₆ alkoxy-C₁₋₆ alkyl;
R₁₀ is selected from the group consisting of hydrogen, C₁₋₆ alkyl, C₃₋₇ cycloalkyl, C₃₋₇ cycloalkyl-C₁₋₆ alkyl, C₂₋₆ alkenyl, hydroxy-C₁₋₆ alkyl, C₁₋₆ alkoxy-C₁₋₆ alkyl, and COR₁₁
wherein R₁₁ is selected from the group consisting of hydrogen, C₁₋₆ alkyl, perhalo-C₁₋₆ alkyl, C₃₋₇ cycloalkyl, C₃₋₇ cycloalkyl-C₁₋₆ alkyl, C₂₋₆ alkenyl, hydroxy-C₁₋₆ alkyl, C₁₋₆ alkoxy, and C₁₋₆ alkoxy-C₁₋₆ alkyl; or
R₉, R₁₀, and nitrogen of B ring form a saturated 3-7 membered heterocyclic ring containing a O, S or NR₁₂,
wherein R₁₂ is selected from the group consisting of hydrogen, C₁₋₆ alkyl, perhalo-C₁₋₆ alkyl, aryl, aryl substituted with one or more groups selected from the group consisting of C₁₋₆ alkyl, halogen, nitro, amino, C₁₋₆ alkylamino, perhalo-C₁₋₆ alkyl, hydroxy-C₁₋₆ alkyl, C₁₋₆ alkoxy, C₁₋₆ alkoxy-C₁₋₆ alkyl and COR₁₃, wherein R₁₃ is selected from the group consisting of hydrogen, C₁₋₆ alkyl, perhalo-C₁₋₆ alkyl, C₁₋₆ alkoxy, aryl, and aryl substituted with one or more of C₁₋₆ alkyl, perhalo-C₁₋₆ alkyl, hydroxy-C₁₋₆ alkyl, and C₁₋₆ alkoxy-C₁₋₆ alkyl;
R₁₁₀-R₁₁₁ are each independently selected from the group consisting of hydrogen; halo, acyl, alkyl, substituted alkyl, alkoxy, substituted alkoxy, alkenyl, alkynyl, cycloalkyl, hydroxyl, cyano, nitro, azido, amido, hydrazine, amino, substituted amino, hydroxycarbonyl, alkoxycarbonyl, alkylcarbonyloxy, alkylcarbonylamino, carbamoyloxy, arylsulfonyloxy, alkylsulfonyloxy, -C(R₁₁₇)=N-(O)ⱼ-R₁₁₈ wherein R₁₁₇ is H, alkyl, alkenyl, cycloalkyl, or aryl, j is 0 or 1, and R₁₁₈ is H, alkyl, alkenyl, cycloalkyl, or heterocycle, R₁₁₉C(O)O- wherein R₁₁₉ is halogen, amino, substituted amino, heterocycle, substituted heterocycle, and R₁₂₀-O-(CH₂)ₖ- where k is an integer of 1-10 and R₁₂₀ is alkyl, phenyl, substituted phenyl, cycloalkyl, substituted cycloalkyl, heterocycle, or substituted heterocycle; or
R₇ together with R₁₁₀ or R₁₁₀ together with R₁₁₁ form substituted or unsubstituted methylenedioxy, ethylenedioxy, or ethyleneoxy; and
R₁₁₂ is H or OR', wherein R' is alkyl, alkenyl, cycloalkyl, haloalkyl, or hydroxyalkyl.

2. A compound according to claim 1 wherein D is a residue of the compound of the Formula (II) wherein R₁₁₀-R₁₁₁ are each independently selected from the group consisting of hydrogen and hydroxyl; and R₁₁₂ is H.

3. The compound of claim 1, wherein the residue of the camptothecin analog is

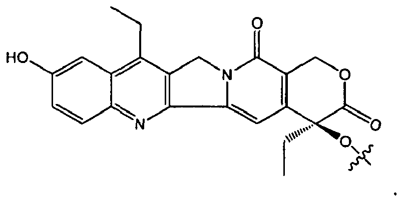

4. The compound of claim 1, wherein L is a residue of glycine, alanine, methionine or sarcosine.

5. The compound of claim 4, wherein L is a residue of glycine.

6. The compound of claim 1, wherein m is 1.

7. The compound of claim 1, wherein n is from 28 to 341, preferably from 114 to 227, more preferably 227 so that the total molecular weight of the polymeric portion of the compound is about 40,000 daltons.

8. The compound of claim 1 having at least about 3.9 loading of (L)ₘ-D.

9. A compound of claim 1, selected from the group consisting of 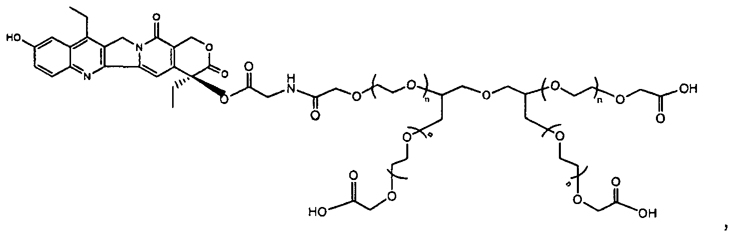 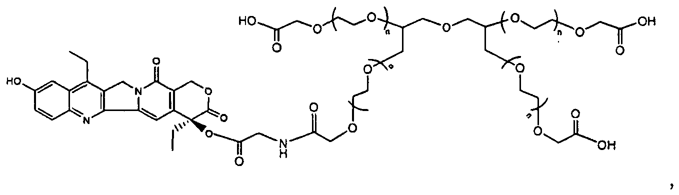 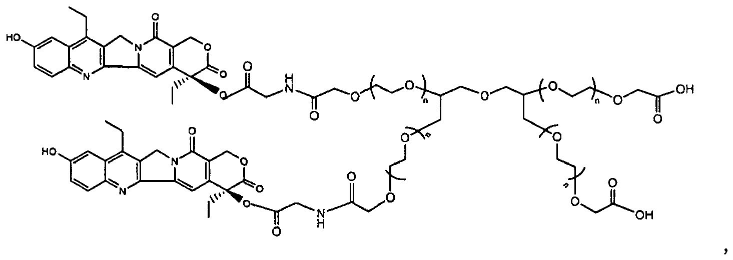 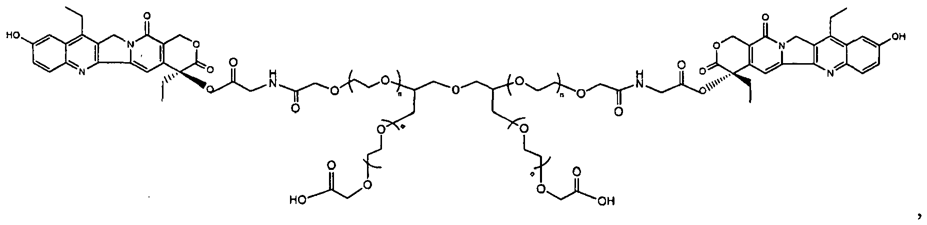 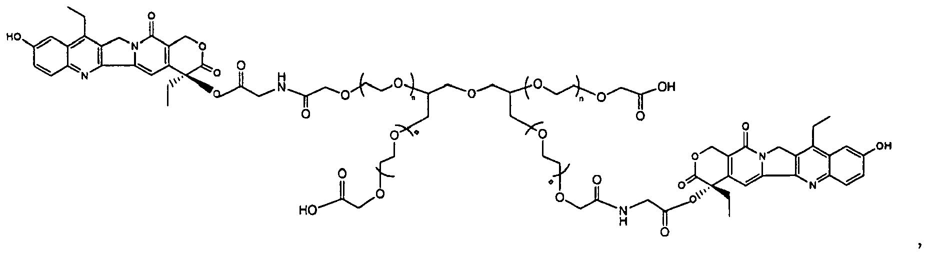 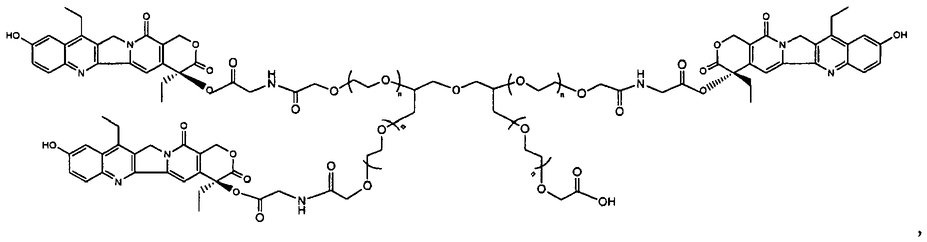 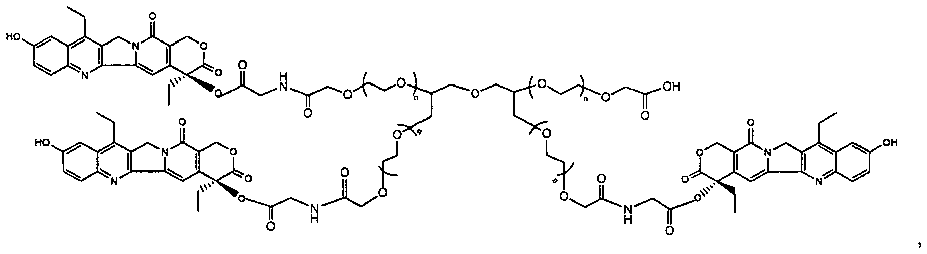 and

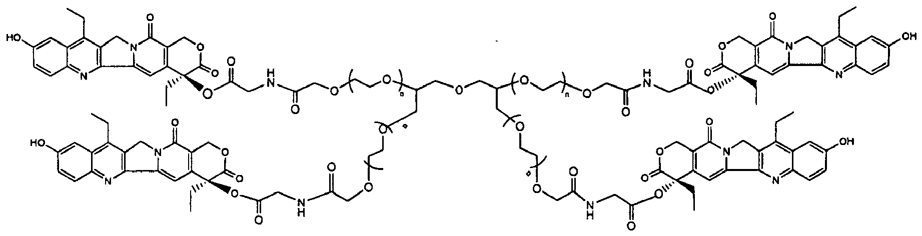

10. The compound of any of the preceding claims for use in a method for treatment of a mammal by therapy.

11. The compound of claim 10 for use for treating cancer, comprising administering an effective amount of compound of claim 1 to a patient in need thereof.

12. The compound for use of claim 11, wherein the cancer is selected from the group consisting of solid tumors, lymphomas, lung cancer, small cell lung cancer, acute lymphocytic leukemia (ALL), breast cancer, colorectal cancer, pancreatic cancer, glioblastoma, ovarian cancer and gastric cancer.

13. The compound for use of claim 11, wherein the cancer is metastatic.

14. The compound for use of claim 11, wherein the cancer is breast cancer, colorectal cancer, lung, ovarian or pancreatic cancer and the compound is

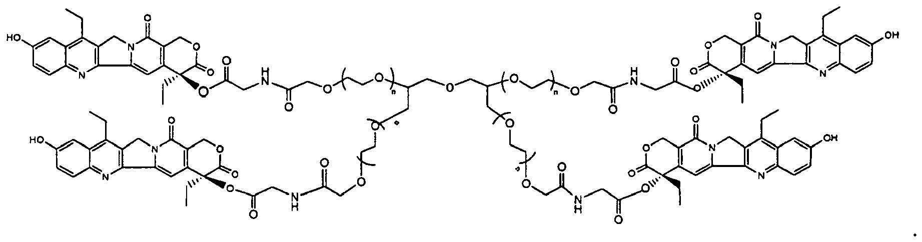

15. The compound for use of claim 12, wherein the compound is administered in amounts of from about 1mg/kg/week to about 100mg/kg/week, preferably from about 2mg/kg/week to about 60mg/kg/week.

16. The compound for use of claim 14, wherein the metastatic disease is breast cancer and the compound is administered in amounts of from about 5 to about 20mg/kg/dose.

17. The compound for use of claim 14, wherein the metastatic disease is colorectal cancer or pancreatic cancer and the compound is administered in amounts of from about 10 to about 30mg/kg/dose.

18. A method of preparing a multi-arm polymeric prodrug, comprising:
(a) providing one equivalent of a camptothecin or camptothecin analog containing an available 20-hydroxyl group and one or more equivalents of a bifunctional linker containing an available carboxylic acid group;
(b) reacting the two reactants under conditions effective to form a camptothecin-bifunctional linker intermediate having an available amine group; and
(c) reacting one or more equivalents per active site of the resulting intermediate and one equivalent of an activated polymer of the formula (I),
wherein the formula (I) is 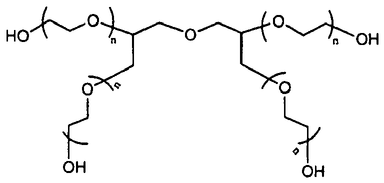 under conditions effective to form the multi-arm polymeric prodrug having the structure of 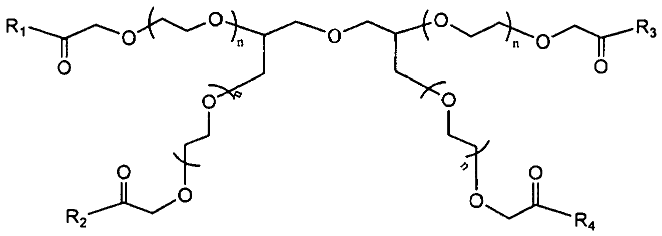 wherein
R₁, R₂, R₃ and R₄ are independently OH or (L)ₘ-D;
L is a bifunctional linker;
m is 0 or a positive integer;
n is a positive integer;
provided that R₁, R₂, R₃ and R₄ are not all OH; and
D is a residue of a camptothecin or of the compound of the Formula (II) 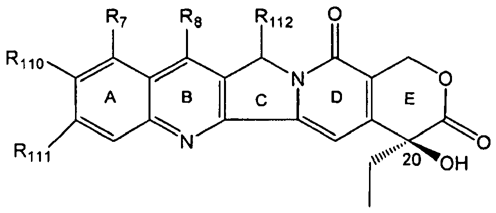 wherein
R₇ is selected from the group consisting of NO₂, NH₂, N₃, hydrogen, halogen, F, Cl, Br, I, COOH, OH, O-C₁₋₈ alkyl, SH, S-C₁₋₃ alkyl, CN, CH₂NH₂, NH-C₁₋₃ alkyl, CH₂-NH-C₁₋₃ alkyl, N(C₁₋₃ alkyl)₂, CH₂N(C₁₋₃alkyl), O-, NH- and S-CH₂CH₂N(CH₂CH₂OH)₂, O-, NH- and S-CH₂CH₂CH₂N(CH₂CH₂OH)₂, O-, NH- and S-CH₂CH₂N(CH₂CH₂CH₂OH)₂, O-, NH- and S-CH₂CH₂CH₂N(CH₂CH₂CH₂OH₂)₂, O-, NH-and S-CH₂CH₂N(C₁₋₃ alkyl)₂, O-, NH- and S-CH₂CH₂CH₂N(C₁₋₃ alkyl)₂, CHO and C₁₋₃ alkyl; and
R₈ is selected from the group consisting of H, C₁₋₈ alkyl and CH₂NR₉R₁₀
wherein
R₉ is selected from the group consisting of hydrogen, C₁₋₆ alkyl, C₃₋₇ cycloalkyl, C₃₋₇ cycloalkyl-C₁₋₆ alkyl, C₂₋₆ alkenyl, hydroxy-C₁₋₆ alkyl, and C₁₋₆ alkoxy-C₁₋₆ alkyl;
R₁₀ is selected from the group consisting of hydrogen, C₁₋₆ alkyl, C₃₋₇ cycloalkyl, C₃₋₇ cycloalkyl-C₁₋₆ alkyl, C₂₋₆ alkenyl, hydroxy-C₁₋₆ alkyl, C₁₋₆ alkoxy-C₁₋₆ alkyl, and COR₁₁
wherein R₁₁ is selected from the group consisting of hydrogen, C₁₋₆ alkyl, perhalo-C₁₋₆ alkyl, C₃₋₇ cycloalkyl, C₃₋₇ cycloalkyl-C₁₋₆ alkyl, C₂₋₆ alkenyl, hydroxy-C₁₋₆ alkyl, C₁₋₆ alkoxy, and C₁₋₆ alkoxy-C₁₋₆ alkyl; or R₉, R₁₀, and nitrogen of B ring form a saturated 3-7 membered heterocyclic ring containing a O, S or NR₁₂,
wherein R₁₂ is selected from the group consisting of hydrogen, C₁₋₆ alkyl, perhalo-C₁₋₆ alkyl, aryl, aryl substituted with one or more groups selected from the group consisting of C₁₋₆ alkyl, halogen, nitro, amino, C₁₋₆ alkylamino, perhalo-C₁₋₆ alkyl, hydroxy-C₁₋₆ alkyl, C₁₋₆ alkoxy, C₁₋₆ alkoxy-C₁₋₆ alkyl and COR₁₃, wherein R₁₃ is selected from the group consisting of hydrogen, C₁₋₆ alkyl, perhalo-C₁₋₆ alkyl, C₁₋₆ alkoxy, aryl, and aryl substituted with one or more of C₁₋₆ alkyl, perhalo-C₁₋₆ alkyl, hydroxy-C₁₋₆ alkyl, and C₁₋₆ alkoxy-C₁₋₆ alkyl;
R₁₁₀-R₁₁₁ are each independently selected from the group consisting of hydrogen; halo, acyl, alkyl, substituted alkyl, alkoxy, substituted alkoxy, alkenyl, alkynyl, cycloalkyl, hydroxyl, cyano, nitro, azido, amido, hydrazine, amino, substituted amino, hydroxycarbonyl, alkoxycarbonyl, alkylcarbonyloxy, alkylcarbonylamino, carbamoyloxy, arylsulfonyloxy, alkylsulfonyloxy, -C(R₁₁₇)=N-(O)ⱼ-R₁₁₈ wherein R₁₁₇ is H, alkyl, alkenyl, cycloalkyl, or aryl, j is 0 or 1, and R₁₁₈ is H, alkyl, alkenyl, cycloalkyl, or heterocycle, R₁₁₉C(O)O- wherein R₁₁₉ is halogen, amino, substituted amino, heterocycle, substituted heterocycle, and R₁₂₀-O-(CH₂)ₖ- where k is an integer of 1-10 and R₁₂₀ is alkyl, phenyl, substituted phenyl, cycloalkyl, substituted cycloalkyl, heterocycle, or substituted heterocycle; or
R₇ together with R₁₁₀ or R₁₁₀ together with R₁₁₁ form substituted or unsubstituted methylenedioxy, ethylenedioxy, or ethyleneoxy; and
R₁₁₂ is H or OR', wherein R' is alkyl, alkenyl, cycloalkyl, haloalkyl, or hydroxyalkyl.

## Patentansprüche

1. Verbindung der Formel (I) 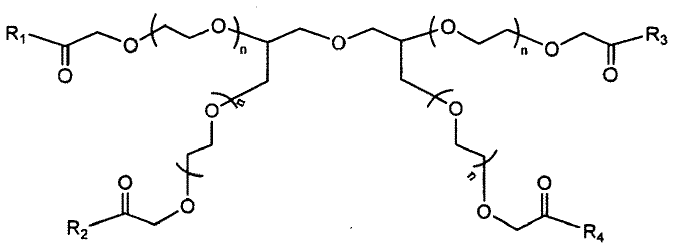 wobei
R₁, R₂, R₃ und R₄ unabhängig OH oder (L)ₘ-D sind;
L ein Rest einer Aminosäure oder ein Aminosäurederivat ist, wobei das Aminosäurederivat ausgewählt ist aus der Gruppe bestehend aus
2-Aminoadipinsäure, 3-Aminoadipinsäure, Beta-Alanin, Beta-Aminopropionsäure, 2-Aminobuttersäure, 4-Aminobuttersäure, Piperidinsäure, 6-Aminocapronsäure, 2-Aminoheptansäure, 2-Aminoisobuttersäure, 3-Aminoisobuttersäure, 2-Aminopimelinsäure, 2,4-Aminobuttersäure, Desmosin, 2,2-Diaminopimelinsäure, 2,3-Diaminopropionsäure, n-Ethylglycin, N-Ethylasparagin, 3-Hydroxyprolin, 4-Hydroxyprolin, Isodesmosin, Allo-Isoleucin, N-Methylglycin, Sarcosin, N-Methylisoleucin, 6-N-Methyllysin, N-Methylvalin, Norvalin, Norleucin und Ornithin; oder
L ein bifunktioneller Linker ist, ausgewählt aus der Gruppe bestehend aus
-[C(O)]ᵥNR₂₁(CR₂₂R₂₃O)ₜ-,
-[C(O)]ᵥNR₂₁(CR₂₂R₂₃O)ₜ(CR₂₄R₂₅)_{y}O-,
-[C(O)]ᵥNR₂₁(CR₂₂R₂₃O)ₜ(CR₂₄R₂₅)_{y}-,
-[C(O)]ᵥNR₂₁(CR₂₂R₂₃O)ₜO-,
-[C(O)]ᵥNR₂₁(CR₂₂R₂₃O)ₜ(CR₂₄R₂₅ O)_{y}NR₂₆-,
-[C(O)]ᵥO(CR₂₂R₂₃)_{t y}NR₂₆-
-[C(O)]ᵥNR₂₁(CR₂₂R₂₃O)ₜO-,
-[C(O)]ᵥO(CR₂₂R₂₃)_{t y}NR₂₆
-[C(O)ᵥNR₂₁(CR₂₂R₂₃O)ₜ(CR₂₄R₂₅ O)_{y}-,
-[C(O)]ᵥNR₂₁(CR₂₂R₂₃O)ₜ(CR₂₄R₂₅)_{y} NR₂₆-, 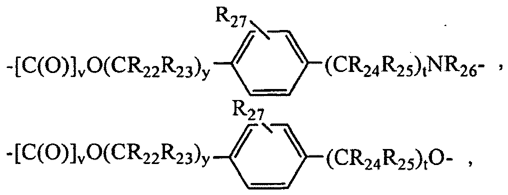 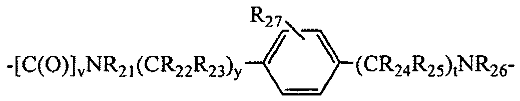
und 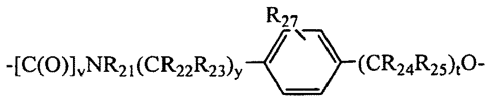 wobei:
R_{21-R26} unabhängig ausgewählt sind aus der Gruppe bestehend aus Wasserstoff, C₁₋₆ Alkylen, C₂₋₆ Alkenylen, C₂₋₆ Alkynylen, C₃₋₁₉ verzweigten Alkylen, C₃₋₈ Cycloalkylen, C₁₋₆ substituierten Alkylen, C₂₋₆ substituierten Alkenylen, C₂₋₆ substituierten Alkynylen, C₃₋₈ substituierten Cycloalkylen, Arylen, substituierten Arylen, Aralkylen, C₁₋₆ Heteroalkylen, substituierten C₁₋₆ Heteroalkylen, C₁₋₆ Alkoxy, Phenoxy und C₁₋₆ Heteroalkoxy;
R₂₇ ausgewählt ist aus der Gruppe bestehend aus Wasserstoff, C₁₋₆ Alkylen, C₂₋₆ Alkenylen, C₂₋₆ Alkynylen, C₃₋₁₉ verzweigten Alkylen, C₃₋₈ Cycloalkylen, C₁₋₆ substituierten Alkylen, C₂₋₆ substituierten Alkenylen, C₂₋₆ substituierten Alkynylen, C₃₋₈ substituierten Cycloalkylen, Arylen, substituierten Arylen, Aralkylen, C₁₋₆ Heteroalkylen, substituierten C₁₋₆ Heteroalkylen, C₁₋₆ Alkoxy, Phenoxy, C₁₋₆ Heteroalkoxy, NO₂, Haloalkyl und Halogen;
t und y individuell ausgewählte positive Ganzzahlen zwischen ungefähr 1 bis ungefähr 4 sind;
und
v 0 oder 1 ist;
m eine positive Ganzzahl von 1 bis 10 ist;
n eine positive Ganzzahl ist;
vorausgesetzt, dass R₁, R₂, R₃ und R₄ nicht alle OH sind; und
D ein Rest eines Camptothecins oder der Verbindung der Formel (II) 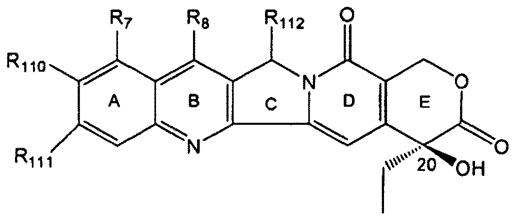
ist,
wobei
R₇ ausgewählt ist aus der Gruppe bestehend aus NO₂, NH₂, N₃, Wasserstoff, Halogen, F, Cl, Br, I, COOH, OH, O-C₁₋₈ Alkyl, SH, S-C₁₋₃ Alkyl, CN, CH₂NH₂, NH-C₁₋₃Alkyl, CH₂-NH-C₁₋₃ Alkyl, N(C₁₋₃ Alkyl)₂, CH₂N(C₁₋₃ Alkyl), O-, NH- und S-CH₂CH₂N(CH₂CH₂OH)₂, O-, NH- und S-CH₂CH₂CH₂N(CH₂CH₂OH)₂, O-, NH- und S- CH₂CH₂N(CH₂CH₂CH₂OH)₂, O-, NH und S-CH₂CH₂CH₂N(CH₂CH₂CH₂OH₂)₂, O-, NH- und S- CH₂CH₂N(C₁₋₃ Alkyl)₂, O-, NH- und S- CH₂CH₂CH₂N(C₁₋₃ Alkyl)₂, CHO und C₁₋₃ Alkyl; und
R₈ ausgewählt ist aus der Gruppe bestehend aus H, C₁₋₈ Alkyl und CH₂NR₉R₁₀,
wobei
R₉ ausgewählt ist aus der Gruppe, bestehend aus Wasserstoff, C₁₋₆ Alkyl, C₃₋₇ Cycloalkyl, C₃₋₇ Cycloalkyl- C₁₋₆ Alkyl, C₂₋₆ Alkenyl, Hydroxy-C₁₋₆ Alkyl und C₁₋₆ Alkoxy- C₁₋₆ Alkyl;
R₁₀ ausgewählt ist aus der Gruppe bestehend aus Wasserstoff, C₁₋₆ Alkyl, C₃₋₇ Cycloalkyl, C₃₋₇ Cycloalkyl- C₁₋₆ Alkyl, C₂₋₆ Alkenyl, Hydroxy-C₁₋₆ Alkyl, C₁₋₆ Alkoxy- C₁₋₆ Alkyl und COR₁₁,
wobei R₁₁ ausgewählt ist aus der Gruppe bestehend aus Wasserstoff, C₁₋₆ Alkyl, Perhalo-C₁₋₆ Alkyl, C₃₋₇ Cycloalkyl, C₃₋₇ Cycloalkyl-C₁₋₆ Alkyl, C₂₋₆ Alkenyl, Hydroxy-C₁₋₆ Alkyl, C₁₋₆ Alkoxy und C₁₋₆ Alkoxy-C₁₋₆ Alkyl; oder
R₉, R₁₀ und Stickstoff von B Ring einen gesättigten 3-7-gliedrigen heterozyklischen Ring, enthaltend ein O, S oder NR₁₂, bilden,
wobei R₁₂ ausgewählt ist aus der Gruppe bestehend aus Wasserstoff, C₁₋₆ Alkyl, Perhalo- C₁₋₆ Alkyl, Aryl, Aryl substituiert mit einer oder mehreren Gruppen, ausgewählt aus der Gruppe bestehend aus C₁₋₆ Alkyl, Halogen, Nitro, Amino, C₁₋₆ Alkylamino, Perhalo- C₁₋₆ Alkyl, Hydroxy-C₁₋₆ Alkyl, C₁₋₆ Alkoxy, C₁₋₆ Alkoxy- C₁₋₆ Alkyl und COR₁₃, wobei R₁₃ ausgewählt ist aus der Gruppe bestehend aus Wasserstoff, C₁₋₆ Alkyl, Perhalo- C₁₋₆ Alkyl, C₁₋₆ Alkoxy, Aryl und Aryl substituiert mit einem oder mehreren von C₁₋₆ Alkyl, Perhalo-C₁₋₆ Alkyl, Hydroxy-C₁₋₆ Alkyl und C₁₋₆ Alkoxy- C₁₋₆ Alkyl;
R₁₁₀-R₁₁₁ unabhängig ausgewählt sind aus der Gruppe bestehend aus Wasserstoff; Halo, Acryl, Alkyl, substituiertem Alkyl, Alkoxy, substituiertem Alkoxy, Alkenyl, Alkynyl, Cycloalkyl, Hydroxyl, Cyano, Nitro, Azido, Amido, Hydrazin, Amino, substituiertem Amino, Hydroxycarbonyl, Alkoxycarbonyl, Alkylcarbonyloxy, Alkylcarbonylamino, Carbamoyloxy, Arylsulfonyloxy, Alkylsulfonyloxy, -C(R₁₁₇)=N-(O)ⱼ-R₁₁₈, wobei R₁₁₇ H, Alkyl, Alkenyl, Cycloalkyl oder Aryl ist, j 0 oder 1 ist und R₁₁₈ H, Alkyl, Alkenyl, Cycloalkyl oder ein Heterozyklus ist, R₁₁₉C(O)O-, wobei R₁₁₉ Halogen, Amino, substituiertes Amino, ein Heterozyklus, ein substituierter Heterozyklus und R₁₂₀-O-(CH₂)ₖ-ist, wobei k eine Ganzzahl von 1-10 ist und R₁₂₀ Alkyl, Phenyl, substituiertes Phenyl, Cycloalkyl, substituiertes Cycloalkyl, ein Heterozyklus oder ein substituierter Heterozyklus ist; oder
R₇ zusammen mit R₁₁₀ oder R₁₁₀ zusammen mit R₁₁₁ substituiertes oder nicht substituiertes Methylendioxy, Ethylendioxy oder Ethylenoxy bilden; und
R₁₁₂ H oder OR' ist, wobei R' Alkyl, Alkenyl, Cycloalkyl, Haloalkyl oder Hydroxyalkyl ist.

2. Verbindung nach Anspruch 1, wobei D ein Rest der Verbindung der Formel (II) ist, wobei R₁₁₀-R₁₁₀ jeweils unabhängig ausgewählt sind aus der Gruppe bestehend aus Wasserstoff und Hydroxyl, und R₁₁₂ H ist.

3. Verbindung nach Anspruch 1, wobei der Rest des Camptothecin-Analogs 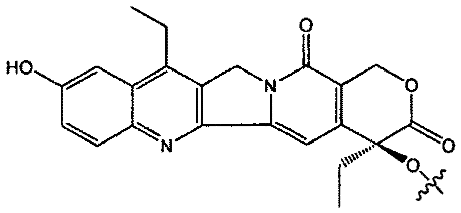 ist.

4. Verbindung nach Anspruch 1, wobei L ein Rest von Glycin, Alanin, Methionin oder Sacrosin ist.

5. Verbindung nach Anspruch 4, wobei L ein Rest von Glycin ist.

6. Verbindung nach Anspruch 1, wobei m 1 ist.

7. Verbindung nach Anspruch 1, wobei n von 28 bis 341, vorzugsweise von 114 bis 227, noch bevorzugter 227 ist, sodass das Gesamtmolekulargewicht des Polymeranteils des Konjugat ungefähr 40.000 Dalton beträgt.

8. Verbindung nach Anspruch 1 mit mindestens ungefähr 3,9 Ladung von (L)ₘ-D.

9. Verbindung nach Anspruch 1, ausgewählt aus der Gruppe bestehend aus 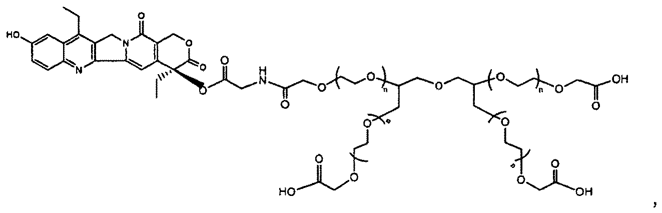 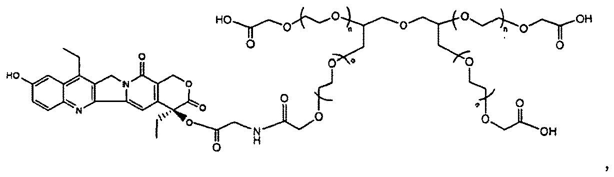 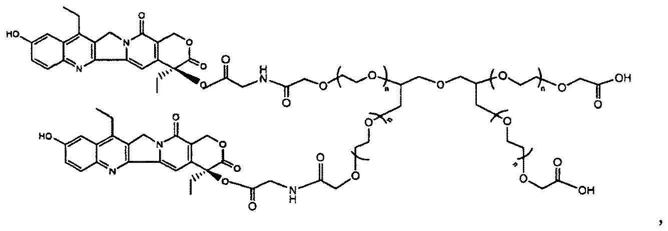 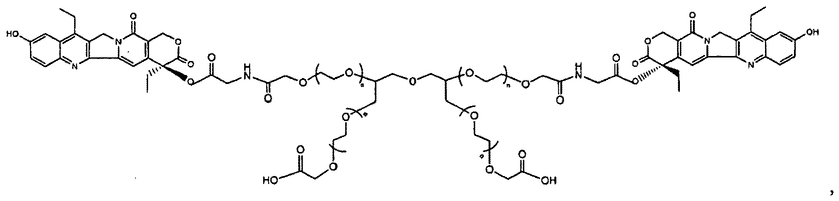 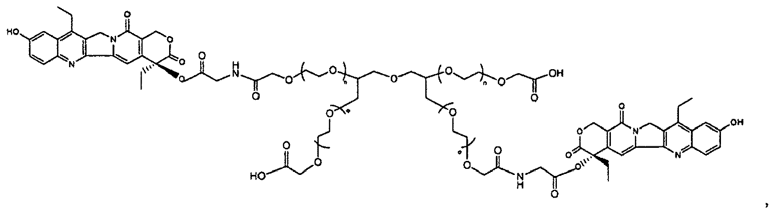 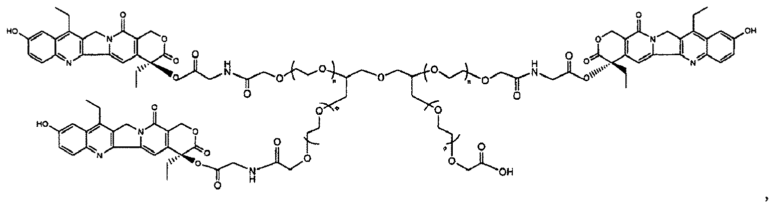 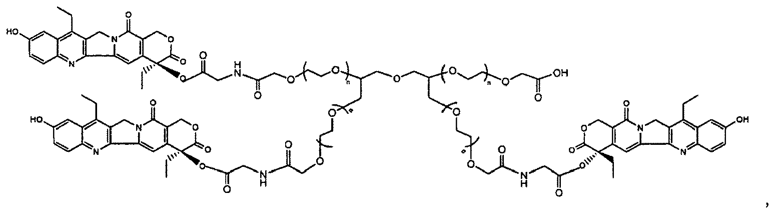 und

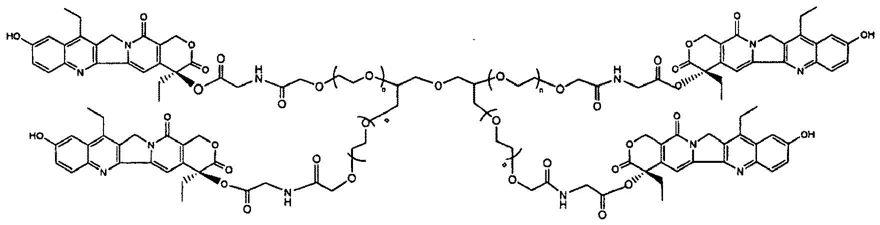

10. Verbindung nach einem der vorhergehenden Ansprüche, zur Verwendung in einem Verfahren zur Behandlung eines Säugers durch Therapie.

11. Verbindung nach Anspruch 10 zur Verwendung für die Behandlung von Krebs, umfassend die Verabreichung einer wirksamen Menge der Verbindung nach Anspruch 1 an einen Patienten, der diese Behandlung benötigt.

12. Verbindung zur Verwendung nach Anspruch 11, wobei der Krebs ausgewählt ist aus der Gruppe, bestehend aus soliden Tumoren, Lymphomen, Lungenkrebs, kleinzelligem Lungenkrebs, akuter lymphozytischer Leukämie (ALL), Brustkrebs, Kolorektalkrebs, Bauchspeicheldrüsenkrebs, Glioblastom, Ovarialkrebs und Magenkrebs.

13. Verbindung zur Verwendung nach Anspruch 11, wobei der Krebs metastatisch ist.

14. Verbindung zur Verwendung nach Anspruch 11, wobei es sich bei dem Krebs um Brust-, Kolorektal-, Lungen-, Ovarial- oder Bauchspeicheldrüsenkrebs handelt und die Verbindung 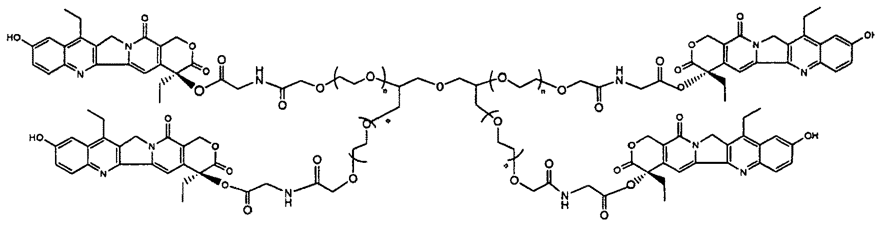 ist.

15. Verbindung zur Verwendung nach Anspruch 12, wobei die Verbindung in Mengen von ungefähr 1 mg/kg/Woche bis ungefähr 100 mg/kg/Woche, vorzugsweise von ungefähr 2 mg/kg/Woche bis ungefähr 60 mg/kg/Woche, verabreicht wird.

16. Verbindung zur Verwendung nach Anspruch 14, wobei es sich bei der metastatischen Erkrankung um Brustkrebs handelt und die Verbindung in Mengen von ungefähr 5 bis ungefähr 20 mg/kg/Dosis verabreicht wird.

17. Verbindung zur Verwendung nach Anspruch 14, wobei es sich bei der metastatischen Erkrankung um Kolorektalkrebs oder Bauchspeicheldrüsenkrebs handelt und die Verbindung in Mengen von ungefähr 10 bis ungefähr 30 mg/kg/Dosis verabreicht wird.

18. Verfahren zur Herstellung eines mehrarmigen polymeren Pro-Pharmakons, umfassend:
(a) die Bereitstellung eines Äquivalents eines Camptothecins oder eines Camptothecin-Analogs, enthaltend eine verfügbare 20-Hydroxyl-Gruppe und eine oder mehrere Äquivalente eines bifunktionellen Linkers, enthaltend eine verfügbare Carboxylsäuregruppe;
(b) das Reagieren der zwei Reaktanten unter Bedingungen, die für die Bildung eines Camptothecin-bifunktionellen Linker-Zwischenstoffs mit einer verfügbaren Aminogruppe wirksam sind; und
(c) das Reagieren von einem oder mehreren Äquivalenten pro aktiver Stelle des resultierenden Zwischenstoffs und oder eines Äquivalents eines aktivierten Polymers der Formel (I),
wobei die Formel (I) 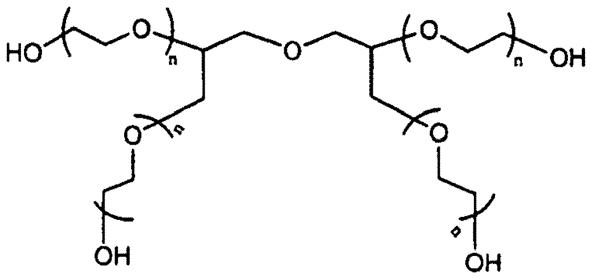 ist,
unter Bedingungen, wirksam für die Bildung des mehrarmigen polymeren Pro-Pharmakons, das die Struktur 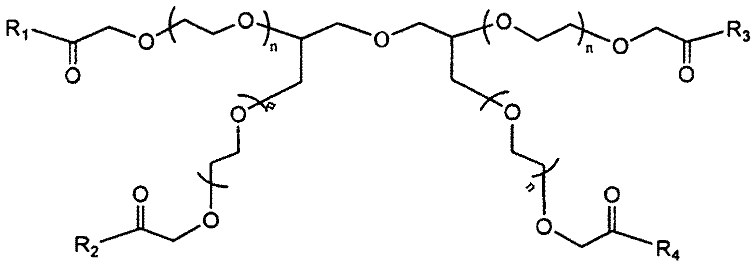 aufweist,
wobei
R₁, R₂, R₃ und R₄ unabhängig OH oder (L)ₘ-D sind;
L ein bifunktioneller Linker ist;
M 0 oder eine positive Ganzzahl ist;
n eine positive Ganzzahl ist;
vorausgesetzt, dass R₁, R₂, R₃ und R₄ nicht alle OH sind; und
D ein Rest eines Camptothecins oder der Verbindung der Formel (II) 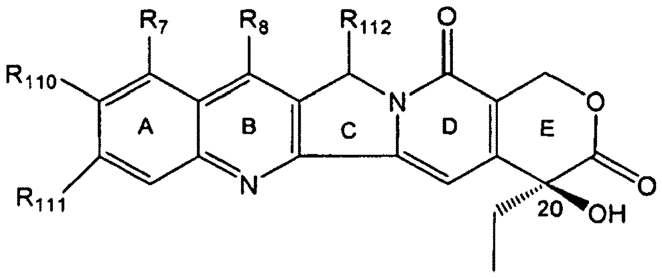 ist,
wobei
R₇ ausgewählt ist aus der Gruppe bestehend aus NO₂, NH₂, N₃, Wasserstoff, Halogen, F, Cl, Br, I, COOH, OH, O-C₁₋₈ Alkyl, SH, S-C₁₋₃ Alkyl, CN, CH₂NH₂, NH-C₁₋₃Alkyl, CH₂-NH-C₁₋₃ Alkyl, N(C₁₋₃ Alkyl)₂, CH₂N(C₁₋₃ Alkyl), O-, NH- und S-CH₂CH₂N(CH₂CH₂OH)₂, O-, NH- und S-CH₂CH₂CH₂N(CH₂CH₂OH)₂, O-, NH- und S- CH₂CH₂N(CH₂CH₂CH₂OH)₂, O-, NH und S- CH₂CH₂CH₂N(CH₂CH₂CH₂OH₂)₂, O-, NH- und S- CH₂CH₂N(C₁₋₃ Alkyl)₂, O-, NH- und S-CH₂CH₂CH₂N(CH₂CH₂CH₂CH₂OH₂)₂, O-, NH- und S-CH₂ CH₂N(C₁₋₃ Alkyl)₂, O-, NH- und S-CH₂CH₂CH₂N(C₁₋₃ Alkyl)₂ und C₁₋₃ Alkyl; und
R₈ ausgewählt ist aus der Gruppe bestehend aus H, C₂₋₈ Alkyl und CH₂NR₉R₁₀,
wobei
R₉ ausgewählt ist aus der Gruppe bestehend aus Wasserstoff, C₁₋₆ Alkyl, C₃₋₇ Cycloalkyl, C₃₋₇ Cycloalkyl-C₁₋₆ Alkyl, C₂₋₆ Alkenyl, Hydroxy-C₁₋₆ Alkyl und C₁₋₆ Alkoxy-C₁₋₆ Alkyl;
R₁₀ ausgewählt ist aus der Gruppe bestehend aus Wasserstoff, C₁₋₆ Alkyl, C₃₋₇ Cycloalkyl, C₃₋₇ Cycloalkyl-C₁₋₆ Alkyl, C₂₋₆ Alkenyl, Hydroxy-C₁₋₆ Alkyl, C₁₋₆ Alkoxy-C₁₋₆ Alkyl und COR₁₁,
wobei R₁₁ ausgewählt ist aus der Gruppe bestehend aus Wasserstoff, C₁₋₆ Alkyl, Perhalo-C₁₋₆ Alkyl, C₃₋₇ Cycloalkyl, C₃₋₇ Cycloalkyl-C₁₋₆ Alkyl, C₂₋₆ Alkenyl, Hydroxy-C₁₋₆ Alkyl, C₁₋₆ Alkoxy und C₁₋₆ Alkoxy-C₁₋₆ Alkyl; oder
R₉, R₁₀ und Stickstoff von B Ring einen gesättigten 3-7-gliedrigen heterozyklischen Ring, enthaltend ein O, S oder NR₁₂, bilden,
wobei R₁₂ ausgewählt ist aus der Gruppe bestehend aus Wasserstoff, C₁₋₆ Alkyl, Perhalo-C₁₋₆ Alkyl, Aryl, Aryl substituiert mit einer oder mehreren Gruppen, ausgewählt aus der Gruppe bestehend aus C₁₋₆ Alkyl, Halogen, Nitro, Amino, C₁₋₆ Alkylamino, Perhalo-C₁₋₆ Alkyl, Hydroxy-C₁₋₆ Alkyl, C₁₋₆ Alkoxy, C₁₋₆ Alkoxy-C₁₋₆ Alkyl und COR₁₃, wobei R₁₃ ausgewählt ist aus der Gruppe bestehend aus Wasserstoff, C₁₋₆ Alkyl, Perhalo-C₁₋₆ Alkyl, C₁₋₆ Alkoxy, Aryl und Aryl substituiert mit einem oder mehreren von C₁₋₆ Alkyl, Perhalo-C₁₆ Alkyl, Hydroxy-C₁₋₆ Alkyl und C₁₋₆ Alkoxy-C₁₋₆ Alkyl;
R₁₁₀-R₁₁₁ unabhängig ausgewählt sind aus der Gruppe bestehend aus Wasserstoff; Halo, Acryl, Alkyl, substituiertem Alkyl, Alkoxy, substituiertem Alkoxy, Alkenyl, Alkynyl, Cycloalkyl, Hydroxyl, Cyano, Nitro, Azido, Amido, Hydrazin, Amino, substituiertem Amino, Hydroxycarbonyl, Alkoxycarbonyl, Alkylcarbonyloxy, Alkylcarbonylamino, Carbamoyloxy, Arylsulfonyloxy, Alkylsulfonyloxy, -C(R₁₁₇)=N-(O)ⱼ-R₁₁₈, wobei R₁₁₇ H, Alkyl, Alkenyl, Cycloalkyl oder Aryl ist, j 0 oder 1 ist und R₁₁₈ H, Alkyl, Alkenyl, Cycloalkyl oder ein Heterozyklus ist, R₁₁₉C(O)O-, wobei R₁₁₉ Halogen, Amino, substituiertes Amino, ein Heterozyklus, ein substituierter Heterozyklus und R₁₂₀-O-(CH₂)ₖ-ist, wobei k eine Ganzzahl von 1-10 ist und R₁₂₀ Alkyl, Phenyl, substituiertes Phenyl, Cycloalkyl, substituiertes Cycloalkyl, ein Heterozyklus oder ein substituierter Heterozyklus ist; oder
R₇ zusammen mit R₁₁₀ oder R₁₁₀ zusammen mit R₁₁₁ substituiertes oder nicht substituiertes Methylendioxy, Ethylendioxy oder Ethylenoxy bilden; und
R₁₁₂ H oder OR' ist, wobei R' Alkyl, Alkenyl, Cycloalkyl, Haloalkyl oder Hydroxyalkyl ist.

## Revendications

1. Composé de formule (I) 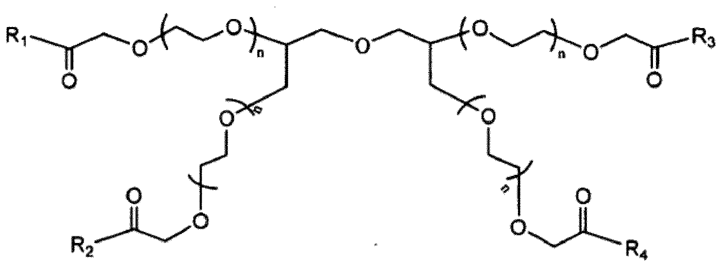 dans lequel
R₁, R₂, R₃ et R₄ sont indépendamment OH ou (L)ₘ-D ;
L est un résidu d'un acide aminé ou dérivé d'acide aminé, le dérivé d'acide aminé étant choisi dans le groupe constitué de
l'acide 2-aminoadipique, l'acide 3-aminoadipique, la bêta-alanine, l'acide bêta-aminopropionique, l'acide 2-aminobutyrique, l'acide 4-aminobutyrique, l'acide pipéridinique, l'acide 6-aminocaproïque, l'acide 2-aminoheptanoïque, l'acide 2-aminoisobutyrique, l'acide 3-aminoisobutyrique, l'acide 2-aminopimélique, l'acide 2,4-aminobutyrique, la desmosine, l'acide 2,2-diaminopimélique, l'acide 2,3-diaminopropionique, la n-éthylglycine, la N-éthylasparagine, la 3-hydroxyproline, la 4-hydroxyproline, l'isodesmosine, l'allo-isoleucine, la N-méthylglycine, la sarcosine, la N-méthyl-isoleucine, la 6-N-méthyl-lysine, la N-méthylvaline, la norvaline, la norleucine, et l'ornithine ; ou
L est un lieur bifonctionnel choisi dans le groupe constitué de
-[C(O)]ᵥNR₂₁(CR₂₂R₂₃O)ₜ-,
-[C(O)]ᵥNR₂₁(CR₂₂R₂₃O)ₜ(CR₂₄R₂₅)_{y}O-,
-[C(O)]ᵥNR₂₁(CR₂₂R₂₃O)ₜ(CR₂₄R₂₅)_{y}-,
-[C(O)]ᵥNR₂₁(CR₂₂R₂₃)ₜO-,
-[C(O)]ᵥNR₂₁(CR₂₂R₂₃)ₜ(CR₂₄CR₂₅O)_{y}NR₂₆-,
-[C(O)]ᵥO(CR₂₂R₂₃)ₜNR₂₆-,
-[C(O)]ᵥO(CR₂₂R₂₃)ₜO-,
-[C(O)]ᵥNR₂₁(CR₂₂R₂₃)ₜNR₂₆-,
-[C(O)]ᵥNR₂₁(CR₂₂R₂₃)ₜ(CR₂₄CR₂₅O)_{y}-,
-[C(O)]ᵥNR₂₁(CR₂₂R₂₃O)ₜ(CR₂₄CR₂₅)_{y}NR₂₆-, 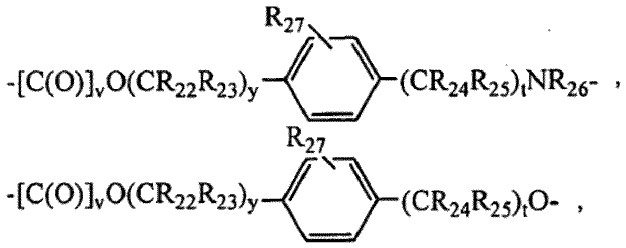 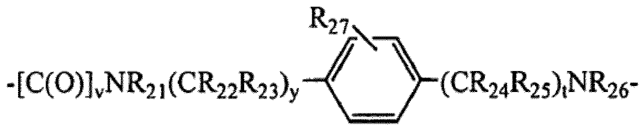
et 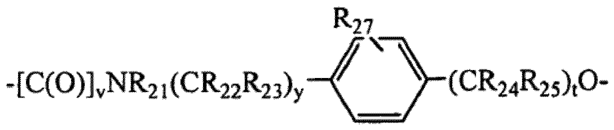 où :
R₂₁ à R₂₆ sont indépendamment choisis dans le groupe constitué d'hydrogène, alkyles en C₁₋₆, alcényles en C₂₋₆, alcynyles en C₂₋₆, alkyles ramifiés en C₃₋₁₉, cycloalkyles en C₃₋₈, alkyles substitués en C₁₋₆, alcényles substitués en C₂₋₆, alcynyles substitués en C₂₋₆, cycloalkyles substitués en C₃₋₈, aryles, aryles substitués, aralkyles, hétéroalkyles en C₁₋₆, hétéroalkyles en C₁₋₆ substitués, alcoxy en C₁₋₆, phénoxy et hétéroalcoxy en C₁₋₆ ;
R₂₇ est choisi dans le groupe constitué d'hydrogène, alkyles en C₁₋₆, alcényles en C₂₋₆, alcynyles en C₂₋₆, alkyles ramifiés en C₃₋₁₉, cycloalkyles en C₃₋₈, alkyles substitués en C₁₋₆, alcényles substitués en C₂₋₆, alcynyles substitués en C₂₋₆, cycloalkyles substitués en C₃₋₈, aryles, aryles substitués, aralkyles, hétéroalkyles en C₁₋₆, hétéroalkyles en C₁₋₆ substitués, alcoxy en C₁₋₆, phénoxy, hétéroalcoxy en C₁₋₆, NO₂, halogénoalkyle et halogène ;
t et y sont individuellement des entiers positifs sélectionnés d'environ 1 à environ 4 ; et
v est 0 ou 1 ;
m est un entier positif de 1 à 10 ;
n est un entier positif ;
à condition que R₁, R₂, R₃ et R₄ ne soient pas tous OH ; et
D est un résidu d'une camptothécine ou du composé de formule (II) 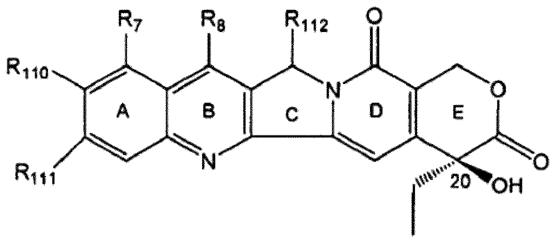 dans lequel
R₇ est choisi dans le groupe constitué de NO₂, NH₂, N₃, hydrogène, halogène, F, Cl, Br, I, COOH, OH, O-(alkyle en C₁₋₈), SH, S-(alkyle en C₁₋₃), CN, CH₂NH₂, NH-(alkyle en C₁₋₃), CH₂-NH-(alkyle en C₁₋₃), N(alkyle en C₁₋₃)₂, CH₂N(alkyle en C₁₋₃), O-, NH- et S-CH₂CH₂N(CH₂CH₂OH)₂, O-, NH- et S-CH₂CH₂CH₂N(CH₂CH₂OH)₂, O-, NH- et S-CH₂CH₂N(CH₂CH₂CH₂OH)₂, O-, NH- et S-CH₂CH₂CH₂N(CH₂CH₂CH₂OH₂)₂, O-, NH- et S-CH₂CH₂N(alkyle en C₁₋₃)₂, O-, NH- et S-CH₂CH₂CH₂N(alkyle en C₁₋₃)₂, CHO et alkyle en C₁₋₃ ; et
R₈ est choisi dans le groupe constitué de H, alkyle en C₁₋₈ et CH₂NR₉R₁₀
où
R₉ est choisi dans le groupe constitué d'hydrogène, alkyle en C₁₋₆, cycloalkyle en C₃₋₇, (cycloalkyle en C₃₋₇)-(alkyle en C₁₋₆), alcényle en C₂₋₆, hydroxy-(alkyle en C₁₋₆), et (alcoxy en C₁₋₆)-(alkyle en C₁₋₆) ;
R₁₀ est choisi dans le groupe constitué d'hydrogène, alkyle en C₁₋₆, cycloalkyle en C₃₋₇, (cycloalkyle en C₃₋₇)-(alkyle en C₁₋₆), alcényle en C₂₋₆, hydroxy-(alkyle en C₁₋₆), (alcoxy en C₁₋₆)-(alkyle en C₁₋₆), et COR₁₁
où R₁₁ est choisi dans le groupe constitué d'hydrogène, alkyle en C₁₋₆, perhalogéno-(alkyle en C₁₋₆), cycloalkyle en C₃₋₇, (cycloalkyle en C₃₋₇)-(alkyle en C₁₋₆), alcényle en C₂₋₆, hydroxy-(alkyle en C₁₋₆), alcoxy en C₁₋₆, et (alcoxy en C₁₋₆)-(alkyle en C₁₋₆) ; ou
R₉, R₁₀, et l'azote du cycle B forment un cycle hétérocyclique de 3 à 7 chaînons saturé contenant un O, S ou NR₁₂,
où R₁₂ est choisi dans le groupe constitué d'hydrogène, alkyle en C₁₋₆, perhalogéno-(alkyle en C₁₋₆), aryle, aryle substitué par un ou plusieurs groupes choisis dans le groupe constitué d'alkyle en C₁₋₆, halogène, nitro, amino, (alkyle en C₁₋₆)amino, perhalogéno-(alkyle en C₁₋₆), hydroxy-(alkyle en C₁₋₆), alcoxy en C₁₋₆, (alcoxy en C₁₋₆)-(alkyle en C₁₋₆) et COR₁₃, où R₁₃ est choisi dans le groupe constitué d'hydrogène, alkyle en C₁₋₆, perhalogéno-(alkyle en C₁₋₆), alcoxy en C₁₋₆, aryle, et aryle substitué par un ou plusieurs parmi alkyle en C₁₋₆, perhalogéno-(alkyle en C₁₋₆), hydroxy-(alkyle en C₁₋₆), et (alcoxy en C₁₋₆)-(alkyle en C₁₋₆) ;
R₁₁₀ à R₁₁₁ sont chacun indépendamment choisis dans le groupe constitué d'hydrogène ; halogéno, acyle, alkyle, alkyle substitué, alcoxy, alcoxy substitué, alcényle, alcynyle, cycloalkyle, hydroxyle, cyano, nitro, azido, amido, hydrazine, amino, amino substitué, hydroxycarbonyle, alcoxycarbonyle, alkylcarbonyloxy, alkylcarbonylamino, carbamoyloxy, arylsulfonyloxy, alkylsulfonyloxy, -C(R₁₁₇)=N-(O)ⱼ-R₁₁₈ où R₁₁₇ est H, alkyle, alcényle, cycloalkyle, ou aryle, j est 0 ou 1, et R₁₁₈ est H, alkyle, alcényle, cycloalkyle, ou hétérocycle, R₁₁₉C(O)O- où R₁₁₉ est halogène, amino, amino substitué, hétérocycle, hétérocycle substitué, et R₁₂₀-O-(CH₂)ₖ- où k est un entier de 1 à 10 et R₁₂₀ est alkyle, phényle, phényle substitué, cycloalkyle, cycloalkyle substitué, hétérocycle, ou hétérocycle substitué ; ou
R₇ conjointement avec R₁₁₀ ou R₁₁₀ conjointement avec R₁₁₁ forment méthylènedioxy, éthylènedioxy, ou éthylèneoxy substitué ou non substitué ; et
R₁₁₂ est H ou OR', où R' est alkyle, alcényle, cycloalkyle, halogénoalkyle, ou hydroxyalkyle.

2. Composé selon la revendication 1 dans lequel D est un résidu du composé de formule (II) dans lequel R₁₁₀ à R₁₁₁ sont chacun indépendamment choisis dans le groupe constitué d'hydrogène et hydroxyle ; et R₁₁₂ est H.

3. Composé de la revendication 1, dans lequel le résidu de l'analogue de camptothécine est

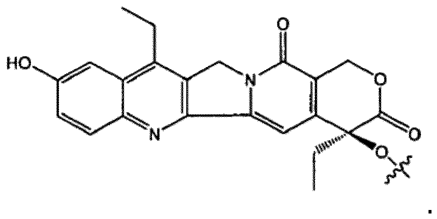

4. Composé de la revendication 1, dans lequel L est un résidu de glycine, alanine, méthionine ou sarcosine.

5. Composé de la revendication 4, dans lequel L est un résidu de glycine.

6. Composé de la revendication 1, dans lequel m est 1.

7. Composé de la revendication 1, dans lequel n est de 28 à 341, de préférence de 114 à 227, plus préférablement 227 de sorte que le poids moléculaire total de la partie polymère du composé soit d'environ 40 000 daltons.

8. Composé de la revendication 1 ayant au moins une charge d'environ 3,9 de (L)ₘ-D.

9. Composé de la revendication 1, choisi dans le groupe constitué de 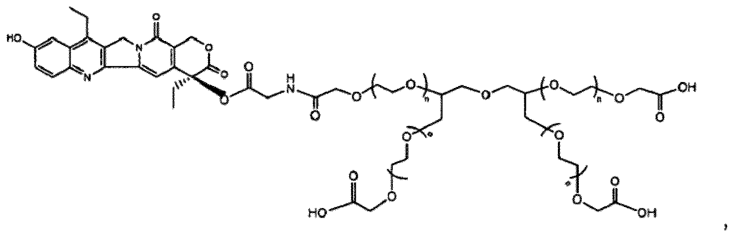 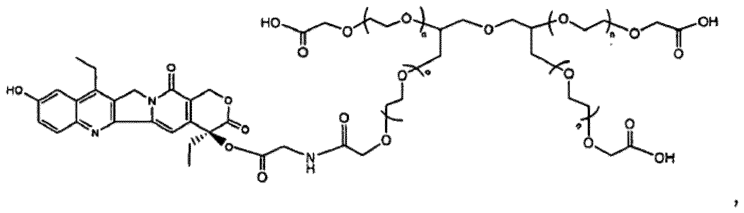 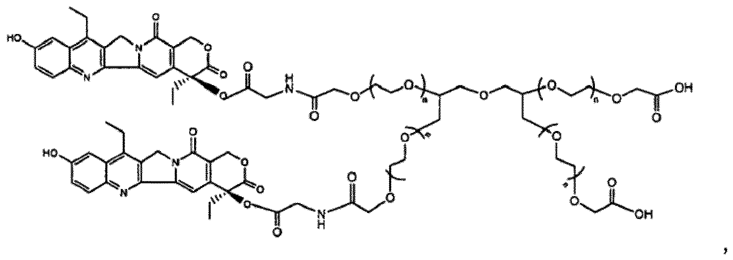 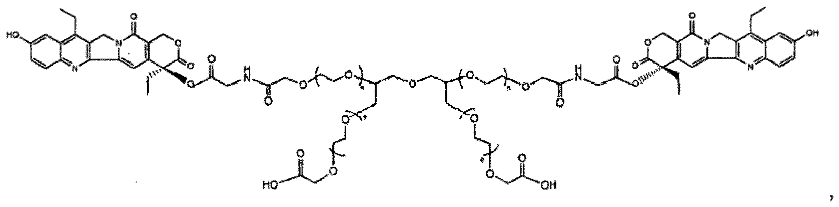 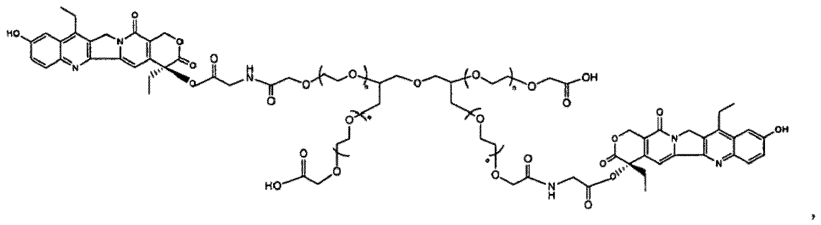 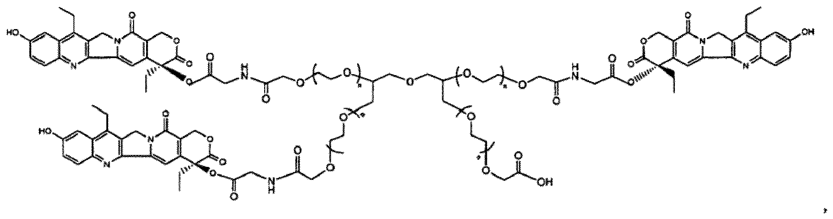 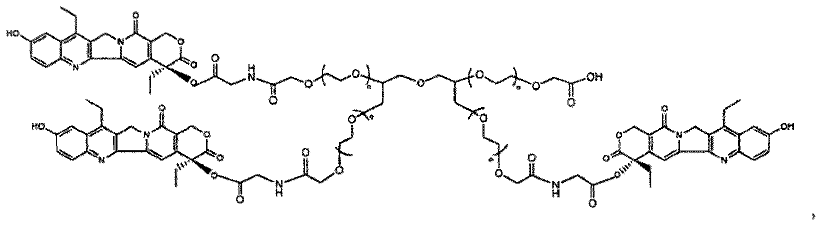 et

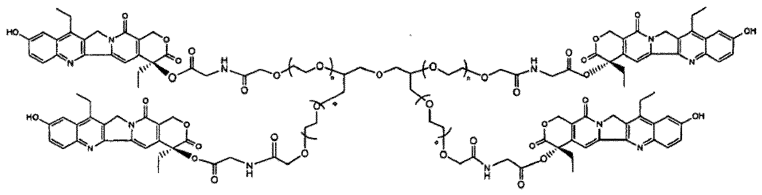

10. Composé de l'une quelconque des revendications précédentes pour utilisation dans un procédé pour le traitement d'un mammifère par thérapie.

11. Composé de la revendication 10 pour utilisation pour traiter le cancer, comprenant l'administration d'une quantité efficace de composé de la revendication 1 à un patient nécessitant celle-ci.

12. Composé pour utilisation de la revendication 11, le cancer étant choisi dans le groupe constitué de tumeurs solides, lymphomes, cancer du poumon, cancer du poumon à petites cellules, leucémie lymphocytaire aiguë (LLA), cancer du sein, cancer colorectal, cancer pancréatique, glioblastome, cancer des ovaires et cancer de l'estomac.

13. Composé pour utilisation de la revendication 11, le cancer étant métastasique.

14. Composé pour utilisation de la revendication 11, le cancer étant le cancer du sein, le cancer colorectal, le cancer du poumon, des ovaires ou pancréatique et le composé est

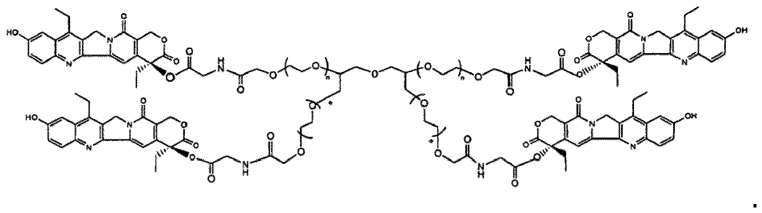

15. Composé pour utilisation de la revendication 12, le composé étant administré en des quantités d'environ 1 mg/kg/semaine à environ 100 mg/kg/semaine, de préférence d'environ 2 mg/kg/semaine à environ 60 mg/kg/semaine.

16. Composé pour utilisation de la revendication 4, la maladie métastasique étant le cancer du sein et le composé étant administré en des quantités d'environ 5 à environ 20 mg/kg/dose.

17. Composé pour utilisation de la revendication 4, la maladie métastasique étant le cancer colorectal ou le cancer pancréatique et le composé étant administré en des quantités d'environ 10 à environ 30 mg/kg/dose.

18. Procédé de préparation d'un promédicament polymère multi-bras, comprenant :
(a) la fourniture d'un équivalent d'une camptothécine ou un analogue de camptothécine contenant un groupe 20-hydroxyle disponible et un ou plusieurs équivalents d'un lieur bifonctionnel contenant un groupe acide carboxylique disponible ;
(b) la réaction des deux réactifs dans des conditions efficaces pour former un intermédiaire bifonctionnel camptothécine-lieur ayant un groupe amine disponible ; et
(c) la réaction d'un ou plusieurs équivalents par site actif de l'intermédiaire résultant et un équivalent d'un polymère activé de formule (I),
la formule (I) étant 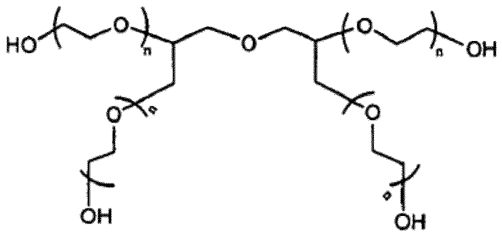 dans des conditions efficaces pour former le promédicament polymère multi-bras ayant la structure de 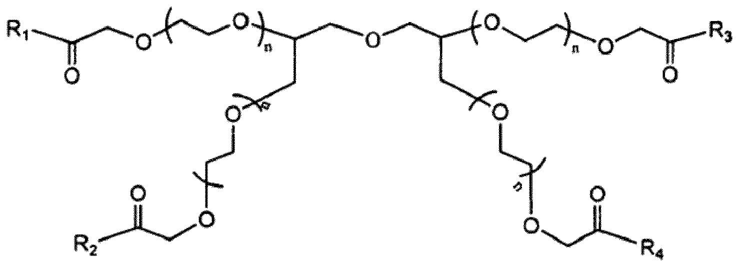 dans laquelle
R₁, R₂, R₃ et R₄ sont indépendamment OH ou (L)ₘ-D ;
L est un lieur bifonctionnel ;
m est 0 ou un entier positif ;
n est un entier positif ;
à condition que R₁, R₂, R₃ et R₄ ne soient pas tous OH ; et
D est un résidu d'une camptothécine ou du composé de formule (II) 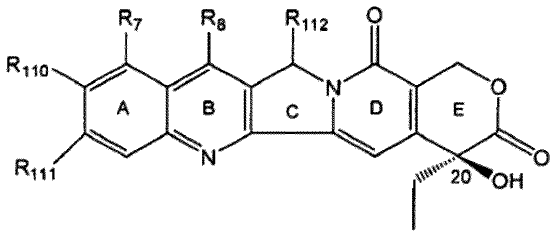 dans lequel
R₇ est choisi dans le groupe constitué de NO₂, NH₂, N₃, hydrogène, halogène, F, Cl, Br, I, COOH, OH, O-(alkyle en C₁₋₈), SH, S-(alkyle en C₁₋₃), CN, CH₂NH₂, NH-(alkyle en C₁₋₃), CH₂-NH-(alkyle en C₁₋₃), N(alkyle en C₁₋₃)₂, CH₂N(alkyle en C₁₋₃), O-, NH- et S-CH₂CH₂N(CH₂CH₂OH)₂, O-, NH- et S-CH₂CH₂CH₂N(CH₂CH₂OH)₂, O-, NH- et S-CH₂CH₂N(CH₂CH₂CH₂OH)₂, O-, NH- et S-CH₂CH₂CH₂N(CH₂CH₂CH₂OH₂)₂, O-, NH- et S-CH₂CH₂N(alkyle en C₁₋₃)₂, O-, NH- et S-CH₂CH₂CH₂N(alkyle en C₁₋₃)₂, CHO et alkyle en C₁₋₃ ; et
R₈ est choisi dans le groupe constitué de H, alkyle en C₁₋₈ et CH₂NR₉R₁₀
où
R₉ est choisi dans le groupe constitué d'hydrogène, alkyle en C₁₋₆, cycloalkyle en C₃₋₇, (cycloalkyle en C₃₋₇)-(alkyle en C₁₋₆), alcényle en C₂₋₆, hydroxy-(alkyle en C₁₋₆), et (alcoxy en C₁₋₆)-(alkyle en C₁₋₆) ;
R₁₀ est choisi dans le groupe constitué d'hydrogène, alkyle en C₁₋₆, cycloalkyle en C₃₋₇, (cycloalkyle en C₃₋₇)-(alkyle en C₁₋₆), alcényle en C₂₋₆, hydroxy-(alkyle en C₁₋₆), (alcoxy en C₁₋₆)-(alkyle en C₁₋₆), et COR₁₁
où R₁₁ est choisi dans le groupe constitué d'hydrogène, alkyle en C₁₋₆, perhalogéno-(alkyle en C₁₋₆), cycloalkyle en C₃₋₇, (cycloalkyle en C₃₋₇)-(alkyle en C₁₋₆), alcényle en C₂₋₆, hydroxy-(alkyle en C₁₋₆), alcoxy en C₁₋₆, et (alcoxy en C₁₋₆)-(alkyle en C₁₋₆) ; ou
R₉, R₁₀, et l'azote du cycle B forment un cycle hétérocyclique de 3 à 7 chaînons saturé contenant un O, S ou NR₁₂,
où R₁₂ est choisi dans le groupe constitué d'hydrogène, alkyle en C₁₋₆, perhalogéno-(alkyle en C₁₋₆), aryle, aryle substitué par un ou plusieurs groupes choisis dans le groupe constitué d'alkyle en C₁₋₆, halogène, nitro, amino, (alkyle en C₁₋₆)amino, perhalogéno-(alkyle en C₁₋₆), hydroxy-(alkyle en C₁₋₆), alcoxy en C₁₋₆, (alcoxy en C₁₋₆)-(alkyle en C₁₋₆) et COR₁₃, où R₁₃ est choisi dans le groupe constitué d'hydrogène, alkyle en C₁₋₆, perhalogéno-(alkyle en C₁₋₆), alcoxy en C₁₋₆, aryle, et aryle substitué par un ou plusieurs parmi alkyle en C₁₋₆, perhalogéno-(alkyle en C₁₋₆), hydroxy-(alkyle en C₁₋₆), et (alcoxy en C₁₋₆)-(alkyle en C₁₋₆) ;
R₁₁₀ à R₁₁₁ sont chacun indépendamment choisis dans le groupe constitué d'hydrogène ; halogéno, acyle, alkyle, alkyle substitué, alcoxy, alcoxy substitué, alcényle, alcynyle, cycloalkyle, hydroxyle, cyano, nitro, azido, amido, hydrazine, amino, amino substitué, hydroxycarbonyle, alcoxycarbonyle, alkylcarbonyloxy, alkylcarbonylamino, carbamoyloxy, arylsulfonyloxy, alkylsulfonyloxy, -C(R₁₁₇)=N-(O)ⱼ-R₁₁₈ où R₁₁₇ est H, alkyle, alcényle, cycloalkyle, ou aryle, j est 0 ou 1, et R₁₁₈ est H, alkyle, alcényle, cycloalkyle, ou hétérocycle, R₁₁₉C(O)O- où R₁₁₉ est halogène, amino, amino substitué, hétérocycle, hétérocycle substitué, et R₁₂₀-O-(CH₂)ₖ- où k est un entier de 1 à 10 et R₁₂₀ est alkyle, phényle, phényle substitué, cycloalkyle, cycloalkyle substitué, hétérocycle, ou hétérocycle substitué ; ou
R₇ conjointement avec R₁₁₀ ou R₁₁₀ conjointement avec R₁₁₁ forment méthylènedioxy, éthylènedioxy, ou éthylèneoxy substitué ou non substitué ; et
R₁₁₂ est H ou OR', où R' est alkyle, alcényle, cycloalkyle, halogénoalkyle, ou hydroxyalkyle.
